# EUROPEAN PATENT APPLICATION

(11) **EP 4 541 373 A1**
(43) Date of publication of application: **23.04.2025**
(21) Application number: 23823979.2
(22) Date of filing: 15.06.2023
(51) Int. Cl.: A61K 45/00, A61P 1/04, A61P 31/04, A61P 37/02, A61P 43/00, A61P 17/02, G01N 33/15

(54) **NOVEL INFLAMMATORY DISEASE TREATMENT AGENT AND SCREENING METHOD FOR SAME**

(30) Priority: 16.06.2022 JP 2022097638
(71) Applicant: OSAKA UNIVERSITY, Suita-shi Osaka 565-0871 (JP); National University Corporation University Of Toyama, Toyama-shi, Toyama 930-8555 (JP)
(72) Inventor: KISHIMOTO Tadamitsu, Suita-shi, Osaka 565-0871 (JP); METWALLY Hozaifa Saad Hassan, Suita-shi, Osaka 565-0871 (JP); OZAWA Tatsuhiko, Toyama-shi, Toyama 930-0194 (JP)
(74) Representative: Witte, Weller & Partner Patentanwälte mbB
(86) International application number: PCT/JP2023/022262
(87) International publication number: WO 2023/243689

(57) **Abstract**

The present invention provides a therapeutic agent for sepsis and/or septic shock, comprising, as an active ingredient, a compound capable of suppressing phosphorylation of threonine at position 749 in human STAT1; a method for screening for a candidate compound serving as an active ingredient of a therapeutic agent for sepsis and/or septic shock, the method comprising selecting a compound capable of suppressing phosphorylation of threonine at position 749 in human STAT1; a therapeutic agent for colitis, comprising, as an active ingredient, a compound capable of promoting phosphorylation of threonine at position 749 in human STAT1; a method for screening for a candidate compound serving as an active ingredient of a therapeutic agent for colitis, the method comprising selecting a compound capable of promoting phosphorylation of threonine at position 749 in human STAT1; a therapeutic agent for systemic lupus erythematosus, comprising, as an active ingredient, a compound capable of inhibiting human STAT1; and a method for screening for a candidate compound serving as an active ingredient of a therapeutic agent for systemic lupus erythematosus, the method comprising selecting a compound capable of inhibiting human STAT1.

## Description

### TECHNICAL FIELD

The present invention relates to a novel therapeutic agent for an inflammatory disease and a screening method for the agent.

### BACKGROUND ART

In multicellular organisms, cells need to recognize extracellular stimuli to modulate various cellular processes including intercellular communications, proliferation, differentiation, homeostasis, and defense against pathogens. Over three decades ago, experiments on the antiviral mechanisms of interferons (IFNs) uncovered the Janus kinase (JAK)-signal transducer and activator of transcription (STAT) pathway, which has since represented a paradigm for membrane-to-nucleus signaling. The canonical JAK-STAT signaling posits that STAT proteins remain latent in the cytoplasm until phosphorylated on a key tyrosine residue within their C-terminal transactivation domain (TAD), which is mediated by a receptor-associated JAK complex.

In mammals, the STAT family comprises seven evolutionary conserved proteins: Stat1, 2, 3, 4, 5a, 5b, and 6. Stat1 is ubiquitously expressed and is composed of a full-length α isoform and its splice variant β isoform, which lacks the last 38 amino acids of its TAD, suggesting a possible contribution of these amino acids to the specificity of the transcriptional activities of Stat1. The JAK-mediated Tyr701 phosphorylation of Stat1 is central for all types of IFN signaling and the transcription of IFN-stimulated genes (ISGs). Several posttranslational modifications such as serine phosphorylation on 708 and 727 contribute to the selectivity of the transcriptional activity of Stat1.

The role of Stat1 in defense against viral infections has been widely studied over decades, but the role of Stat1 in other immunological scenarios, in particular, its role against inflammatory stimuli has not yet been well understood. The inventors have revealed that threonine 749 is a potential phosphorylation site of STAT1 TAD activated by IKK-related kinase β (IKKβ) in lipopolysaccharide (LPS)-stimulated human macrophages. Endocytosis of toll-like receptor 4 (TLR4) led to the noncanonical phosphorylation of STAT1, which contributed to the production of proinflammatory cytokines independently of the canonical JAK-Stat1 pathway (Non Patent Literature 1). However, the biological significance of this noncanonical phosphorylation or its contribution to Stat1 activities has not yet been clarified.

### CITATION LIST

### NON PATENT LITERATURE

Non Patent Literature 1: SCIENCE SIGNALING, 24 Mar 2020, Vol 13, Issue 624. DOI: 10_1126/scisignal_aay0574.

### SUMMARY OF INVENTION

### TECHNICAL PROBLEM

An object of the present invention is to provide a novel therapeutic agent for an inflammatory disease and a screening method for the agent. Specifically, objects of the present invention are to provide a novel therapeutic agent for sepsis and a screening method for the agent; a novel therapeutic agent for colitis and a screening method for the agent; and a novel therapeutic agent for systemic lupus erythematosus and a screening method for the agent. Other objects of the present invention are to provide an antibody capable of binding to human STAT1 phosphorylated at threonine at position 749, and an antibody capable of binding to mouse STAT1 phosphorylated at threonine at position 748.

### SOLUTION TO PROBLEM

The present invention was made to solve the above problems and includes the following.
[A1] A therapeutic agent for sepsis and/or septic shock, comprising, as an active ingredient, a compound capable of suppressing phosphorylation of threonine at position 749 in human STAT1.
[A2] The therapeutic agent according to [A1], wherein the compound is a compound capable of inhibiting a protein kinase that phosphorylates threonine at position 749 in human STAT1.
[A3] The therapeutic agent according to [A1], wherein the compound is a compound capable of inhibiting binding between human STAT1 and a protein kinase that phosphorylates threonine at position 749 in human STAT1.
[A4] The therapeutic agent according to [A1], wherein the compound is a compound capable of specifically binding to threonine at position 749 in human STAT1.
[A5] The therapeutic agent according to [A2] or [A3], wherein the protein kinase is human TBK1 and/or human IKKβ.
[A6] A therapeutic agent for sepsis and/or septic shock, comprising, as an active ingredient, a compound capable of inhibiting human STAT1.
[A7] The therapeutic agent according to [A6], wherein the compound is a compound capable of inhibiting a function of human STAT1.
[A8] The therapeutic agent according to [A6], wherein the compound is a compound capable of inhibiting expression of human STAT1.
[A9] A method for screening for a candidate compound serving as an active ingredient of a therapeutic agent for sepsis and/or septic shock, the method comprising selecting a compound capable of suppressing phosphorylation of threonine at position 749 in human STAT1.
[A10] The method according to [A9], wherein the compound is a compound capable of inhibiting a protein kinase that phosphorylates threonine at position 749 in human STAT1.
[A11] The method according to [A10], wherein the method comprises
   (a) measuring phosphorylation of human STAT1 by the protein kinase that phosphorylates threonine at position 749 in human STAT1 in the presence or absence of a test compound, and
   (b) selecting the test compound as the candidate compound when the phosphorylation of human STAT1 is suppressed in the presence of the test compound as compared to that in the absence of the test compound.
[A12] The method according to [A9], wherein the compound is a compound capable of inhibiting binding between human STAT1 and a protein kinase that phosphorylates threonine at position 749 in human STAT1.
[A13] The method according to [A12], wherein the method comprises
   (a) measuring binding between human STAT1 and the protein kinase that phosphorylates threonine at position 749 in human STAT1 in the presence or absence of a test compound, and
   (b) selecting the test compound as the candidate compound when the binding between human STAT1 and the protein kinase is suppressed in the presence of the test compound as compared to that in the absence of the test compound.
[A14] The method according to any one of [A10] to [A13], wherein the protein kinase is human TBK1 and/or human IKKβ.
[A15] The method according to [A9], wherein the compound is a compound capable of specifically binding to threonine at position 749 in human STAT1.
[A16] The method according to [A15], wherein the method comprises
   (a) contacting a test compound with wild-type human STAT1 and with mutant human STAT1 having replacement of threonine at position 749 by alanine, and measuring an amount of the test compound bound to either STAT1, and
   (b) selecting the test compound as the candidate compound when the amount of the test compound bound to the mutant human STAT1 is smaller than that bound to the wild-type human STAT1.
[A17] A method for screening for a candidate compound serving as an active ingredient of a therapeutic agent for sepsis and/or septic shock, the method comprising selecting a compound capable of inhibiting human STAT1.
[A18] The method according to [A17], wherein the compound is a compound capable of inhibiting a function of human STAT1.
[A19] The method according to [A18], wherein the method comprises
   (a) measuring a function of human STAT1 in the presence or absence of a test compound, and
   (b) selecting the test compound as the candidate compound when the function of human STAT1 is inhibited in the presence of the test compound as compared to that in the absence of the test compound.
[A20] The method according to [A17], wherein the compound is a compound capable of inhibiting expression of human STAT1.
[A21] The method according to [A20], wherein the method comprises
   (a) measuring expression of human STAT1 in the presence or absence of a test compound, and
   (b) selecting the test compound as the candidate compound when the expression of human STAT1 is inhibited in the presence of the test compound as compared to that in the absence of the test compound.
[A22] The method according to [A9] or [A18], wherein the test compound is a cyclic polypeptide.
[A23] A method for treating sepsis and/or septic shock, comprising administering a compound capable of inhibiting phosphorylation of threonine at position 749 in human STAT1.
[A24] A compound capable of inhibiting phosphorylation of threonine at position 749 in human STAT1, for use in treatment of sepsis and/or septic shock.
[A25] Use of a compound capable of inhibiting phosphorylation of threonine at position 749 in human STAT1 in the production of a medicament for treating sepsis and/or septic shock.
[A26] A method for treating sepsis and/or septic shock, comprising administering a compound capable of inhibiting human STAT1.
[A27] A compound capable of inhibiting human STAT1, for use in treatment of sepsis and/or septic shock.
[A28] Use of a compound capable of inhibiting human STAT1 in the production of a medicament for treating sepsis and/or septic shock.
[B1] A therapeutic agent for colitis, comprising, as an active ingredient, a compound capable of promoting phosphorylation of threonine at position 749 in human STAT1.
[B2] The therapeutic agent according to [B1], wherein the compound is a compound capable of activating a protein kinase that phosphorylates threonine at position 749 in human STAT1.
[B3] The therapeutic agent according to [B1], wherein the compound is a compound capable of promoting binding between human STAT1 and a protein kinase that phosphorylates threonine at position 749 in human STAT1.
[B4] The therapeutic agent according to [B2] or [B3], wherein the protein kinase is human TBK1 and/or human IKKβ.
[B5] The therapeutic agent according to any one of [B1] to [B4], wherein the colitis is one or more diseases selected from the group consisting of inflammatory bowel disease, ulcerative colitis, Crohn disease, collagenous colitis, lymphocytic colitis, ischemic colitis, diversion colitis, Behcet disease, infectious colitis, and indeterminate colitis.
[B6] A method for screening for a candidate compound serving as an active ingredient of a therapeutic agent for colitis, the method comprising selecting a compound capable of promoting phosphorylation of threonine at position 749 in human STAT1.
[B7] The method according to [B6], wherein the compound is a compound capable of activating a protein kinase that phosphorylates threonine at position 749 in human STAT1.
[B8] The method according to [B7], wherein the method comprises
   (a) measuring phosphorylation of human STAT1 by the protein kinase that phosphorylates threonine at position 749 in human STAT1 in the presence or absence of a test compound, and
   (b) selecting the test compound as the candidate compound when the phosphorylation of human STAT1 is promoted in the presence of the test compound as compared to that in the absence of the test compound.
[B9] The method according to [B6], wherein the compound is a compound capable of promoting binding between human STAT1 and a protein kinase that phosphorylates threonine at position 749 in human STAT1.
[B10] The method according to [B9], wherein the method comprises
   (a) measuring binding between human STAT1 and the protein kinase that phosphorylates threonine at position 749 in human STAT1 in the presence or absence of a test compound, and
   (b) selecting the test compound as the candidate compound when the binding between human STAT1 and the protein kinase is promoted in the presence of the test compound as compared to that in the absence of the test compound.
[B11] The method according to any one of [B7] to [B10], wherein the protein kinase is human TBK1 and/or human IKKβ.
[B12] The method according to any one of [B6] to [B11], wherein the colitis is one or more diseases selected from the group consisting of inflammatory bowel disease, ulcerative colitis, Crohn disease, collagenous colitis, lymphocytic colitis, ischemic colitis, diversion colitis, Behcet disease, infectious colitis, and indeterminate colitis.
[B13] The method to according to [B6], wherein the test compound is a cyclic polypeptide.
[B14] A method for treating colitis, comprising administering a compound capable of promoting phosphorylation of threonine at position 749 in human STAT1.
[B15] A compound capable of promoting phosphorylation of threonine at position 749 in human STAT1, for use in treatment of colitis.
[B16] Use of a compound capable of promoting phosphorylation of threonine at position 749 in human STAT1 in the production of a medicament for treating colitis.
[C1] A therapeutic agent for systemic lupus erythematosus, comprising, as an active ingredient, a compound capable of inhibiting human STAT1.
[C2] The therapeutic agent according to [C1], wherein the compound is a compound capable of inhibiting a function of human STAT1.
[C3] The therapeutic agent according to [C1], wherein the compound is a compound capable of inhibiting expression of human STAT1.
[C4] A method for screening for a candidate compound serving as an active ingredient of a therapeutic agent for systemic lupus erythematosus, the method comprising selecting a compound capable of inhibiting human STAT1.
[C5] The method according to [C4], wherein the compound is a compound capable of inhibiting a function of human STAT1.
[C6] The method according to [C5], wherein the method comprises
   (a) measuring a function of human STAT1 in the presence or absence of a test compound, and
   (b) selecting the test compound as the candidate compound when the function of human STAT1 is inhibited in the presence of the test compound as compared to that in the absence of the test compound.
[C7] The method according to [C4], wherein the compound is a compound capable of inhibiting expression of human STAT1.
[C8] The method according to [C7], wherein the method comprises
   (a) measuring expression of human STAT1 in the presence or absence of a test compound, and
   (b) selecting the test compound as the candidate compound when the expression of human STAT1 is inhibited in the presence of the test compound as compared to that in the absence of the test compound.
[C9] The method according to [C6] or [C8], wherein the measuring is performed using cells expressing human STAT1.
[C10] The method to according to [C4], wherein the test compound is a cyclic polypeptide.
[C11] A method for treating systemic lupus erythematosus, comprising administering a compound capable of inhibiting human STAT1.
[C12] A compound capable of inhibiting human STAT1 for use in treatment of systemic lupus erythematosus.
[C13] Use of a compound capable of inhibiting human STAT1 in the production of a medicament for treating systemic lupus erythematosus.
[D1] An antibody capable of binding to STAT1 phosphorylated at threonine at the second position from the C-terminus, wherein the antibody has higher binding affinity to STAT1 phosphorylated at the threonine than to wild-type STAT1.
[D2] An antibody capable of binding to human STAT1 phosphorylated at threonine at position 749, wherein the antibody has higher binding affinity to human STAT1 phosphorylated at threonine at position 749 than to wild-type human STAT1.
[D3] An antibody capable of binding to mouse STAT1 phosphorylated at threonine at position 748, wherein the antibody has higher binding affinity to mouse STAT1 phosphorylated at threonine at position 748 than to wild-type mouse STAT1.
[D4] The antibody according to any one of [D1] to [D3], wherein the antibody is a rabbit monoclonal antibody.
[D5] A method for screening for a candidate compound serving as an active ingredient of a therapeutic agent for sepsis and/or septic shock, the method comprising
   (a) measuring phosphorylation of threonine at position 749 in human STAT1 using the antibody according to [D1] or [D2] in the presence or absence of a test compound, and
   (b) selecting the test compound as the candidate compound when the phosphorylation of human STAT1 is suppressed in the presence of the test compound as compared to that in the absence of the test compound.
[D6] A method for screening for a candidate compound serving as an active ingredient of a therapeutic agent for colitis, the method comprising
   (a) measuring phosphorylation of threonine at position 749 in human STAT1 using the antibody according to [D1] or [D2] in the presence or absence of a test compound, and
   (b) selecting the test compound as the candidate compound when the phosphorylation of human STAT1 is promoted in the presence of the test compound as compared to that in the absence of the test compound.
[E1] A genetically modified human cell in which a gene encoding mutant human STAT1 having replacement of threonine at position 749 by alanine is knocked-in.
[E2] A genetically modified mouse cell in which a gene encoding mutant mouse STAT1 having replacement of threonine at position 748 by alanine is knocked-in.
[E3] A method for screening for a candidate compound serving as an active ingredient of a therapeutic agent for sepsis and/or septic shock, the method comprising
   (a) providing
      (1) human cells expressing wild-type human STAT1, and
      (2) human cells generated by knocking-in a gene encoding mutant human STAT1 having replacement of threonine at position 749 by alanine into the cells as defined in (1),
   (b) contacting a test compound with both of the cells as defined in (1) and (2), and measuring the phosphorylation state of the human STAT1 in both of the cells as defined in (1) and (2), and
   (c) selecting the test compound as the candidate compound when the phosphorylation state of the human STAT1 in the cells as defined in (1) is comparable to that in the cells as defined in (2).

### ADVANTAGEOUS EFFECTS OF INVENTION

The present invention provides a novel therapeutic agent for sepsis and a screening method for the agent; a novel therapeutic agent for colitis and a screening method for the agent; and a novel therapeutic agent for systemic lupus erythematosus and a screening method for the agent. The present invention also provides an antibody capable of binding to human STAT1 phosphorylated at threonine at position 749, and an antibody capable of binding to mouse STAT1 phosphorylated at threonine at position 748.

### BRIEF DESCRIPTION OF DRAWINGS

Fig. 1 shows the survival of mice after intraperitoneal administration of LPS (12.5 mg/kg) to Wt/Wt, Wt/Stat1T748A and Stat1T748A/Stat1T748A littermate mice generated by mating Wt/Stat1T748A mice.
Fig. 2 shows the survival of mice after intraperitoneal administration of LPS (12.5 mg/kg) to Stat1KO/Stat1KO and Stat1T748A littermate mice generated by mating Stat1KO/Stat1T748A mice.
Fig. 3 shows the Western blotting detection of expression of endogenous STAT-related proteins in spleens from Wt/Wt mice and Stat1T748A/Stat1T748A mice (littermates) that did not receive LPS.
Fig. 4 shows charts showing the evaluation of acute colitis induced by administering dextran sulfate sodium (DSS) in drinking water to Wt/Wt mice and Stat1T748A/Stat1T748A mice (littermates) for 7 days. Panel (A) shows the weight change during the DSS administration period. Panel (B) shows the body weight on day 7. Panel (C) shows the colon length. Panel (D) shows the severity score of colitis in histopathological test of colon.
Fig. 5 shows charts showing the evaluation of acute colitis induced by administering dextran sulfate sodium (DSS) in drinking water to Stat1KO/Stat1KO mice and Stat1T748A mice (littermates) for 7 days. Panel (A) shows the weight change during the DSS administration period. Panel (B) shows the body weight on day 7. Panel (C) shows the colon length. Panel (D) shows the severity score of colitis in histopathological test of colon.
Fig. 6 shows charts and images showing the evaluation of lupus conditions in Wt/Wt mice and Stat1T748A/Stat1T748A mice (littermates) monitored for 6 months after intraperitoneal administration of 0.5 ml of pristane. Panel (A) shows the survival for 6 months after pristane administration. Panel (B) shows the serum anti-dsDNA titers 6 months after pristane administration. Panel (C) shows microphotographs of the hematoxylin and eosin specimens of kidney sections.
Fig. 7 shows charts and images showing the evaluation of lupus conditions in Stat1KO/Stat1KO mice and Stat1T748A mice (littermates) monitored for 6 months after intraperitoneal administration of 0.5 ml of pristane. Panel (A) shows the survival for 6 months after pristane administration. Panel (B) shows the serum anti-dsDNA titers 6 months after pristane administration. Panel (C) shows microphotographs of the hematoxylin and eosin specimens of kidney sections.
Fig. 8 shows the urinary protein levels in Wt/Wt mice, Stat1T748A/Stat1T748A mice and Stat1KO/Stat1KO mice measured 6 months after intraperitoneal administration of 0.5 ml of pristane or no administration of pristane.
Fig. 9 shows the reactivity of phosphorylation-specific monoclonal antibodies to mouse STAT1 peptide phosphorylated at threonine at position 748 (A), and the reactivity of phosphorylation-specific monoclonal antibodies to human STAT1 peptide phosphorylated at threonine at position 749 (B), as analyzed by ELISA.
Fig. 10 shows the phosphorylation selectivity and cross-reactivity of serial dilution samples of monoclonal antibodies produced from the purified clones selected from the reactivity analysis in Fig. 9, as analyzed by ELISA.
Fig. 11 shows the separation by non-reducing/reducing electrophoresis of monoclonal antibodies from culture supernatants produced by HEK293T cells to which γ and κ chain expression vectors encoding the selected whole antibody molecules were co-introduced.
Fig. 12 shows the detection of mouse STAT1 phosphorylated at threonine at position 748 and human STAT1 phosphorylated at threonine at position 749 by monoclonal antibodies prepared by introducing a human or mouse wild-type STAT1 expression vector or mutant STAT1T748A expression vector in conjunction with an IKKβ expression vector into HEK293T cells.

### DESCRIPTION OF EMBODIMENTS

### Therapeutic agent for inflammatory diseases

The present invention provides a therapeutic agent for an inflammatory disease. The inflammatory disease to be treated with the therapeutic agent of the present invention may be any inflammatory disease whose conditions are manifested and aggravated under influence of phosphorylation of threonine at position 749 in human STAT1 (hereafter referred to as "threonine 749"). Such an inflammatory disease to be treated with the therapeutic agent of the present invention may include, for example, sepsis, septic shock, colitis, systemic lupus erythematosus, etc.

Sepsis is a disease characterized by Systemic Inflammatory Response Syndrome (SIRS) caused by pathogens. Sepsis can present with symptoms including fever, tachypnea, tachycardia, and leukocytosis, and when progressed to the advanced stage, can cause circulatory failure, such as consciousness disorder and pressure drop, as well as organ dysfunction, including dysfunction of the kidney, lung, liver, and coagulation. When sepsis becomes more serious, it will be associated with Acute Respiratory Distress Syndrome (ARDS) or Disseminated Intravascular Coagulation (DIC), and cause septic shock leading to cardiac arrest and death. When sepsis is progressed to a more advanced stage, the prognosis will be worse and mortality will be higher. Early diagnosis and early therapy as possible are required at an earlier stage.

Septic shock is a more severe form of sepsis, and is associated with organ hypoperfusion and hypotonia that are hardly responsive to initial fluid resuscitation. Most of the cases are caused by gram-negative bacillus or gram-positive coccus through hospital infections. Septic shock often emerges in patients with immunodeficiency or chronic and wasting diseases, and its mortality is about 40% on average.

Colitis herein may be any inflammatory disease of the large intestine, and may include, for example, inflammatory bowel disease, ulcerative colitis, Crohn disease, collagenous colitis, lymphocytic colitis, ischemic colitis, diversion colitis, Behcet disease, infectious colitis, indeterminate colitis, etc.

Systemic lupus erythematosus (SLE) is a systemic autoimmune disease caused by inflammatory reactions induced by deposition of antinuclear antibodies or anti-DNA antibodies or their immunocomplexes in various organs and tissues. Deposition of antinuclear antibodies or anti-DNA antibodies and their immunocomplexes on the renal glomeruli may induce lupus nephritis.

The therapeutic agent of the present invention may include a compound capable of suppressing phosphorylation of threonine 749 in human STAT1, a compound capable of promoting phosphorylation of threonine 749 in human STAT1, a compound capable of inhibiting human STAT1, etc. The amino acid sequence of human STAT1 may be, for example, the amino acid sequence of NCBI accession No. NP_009330.1 (SEQ ID NO: 1). The nucleotide sequence encoding the human STAT1 may be, for example, the nucleotide sequence of NCBI accession No. NM_007315.4 (SEQ ID NO: 2).

### (1) Compound capable of suppressing phosphorylation of threonine 749 in human STAT1

The compound capable of suppressing phosphorylation of threonine 749 in human STAT1 may be a compound capable of inhibiting a protein kinase that phosphorylates threonine 749 in human STAT1, or a compound capable of inhibiting binding between human STAT1 and a protein kinase that phosphorylates threonine 749 in human STAT1. The compound capable of inhibiting a protein kinase that phosphorylates threonine 749 in human STAT1 may be a compound capable of inhibiting expression of the protein kinase, or a compound capable of inhibiting a function of the protein kinase. The compound capable of inhibiting binding between human STAT1 and a protein kinase that phosphorylates threonine 749 in human STAT1 may be a compound capable of binding to the protein kinase, thereby inhibiting binding between human STAT1 and the protein kinase, or a compound capable of binding to human STAT1 in a competitive manner with the protein kinase, thereby inhibiting binding between human STAT1 and the protein kinase.

The protein kinase that phosphorylates threonine 749 in human STAT1 may be human TBK1 (TANK Binding Kinase 1) or human IKKβ (I-kappa-B-kinase beta), or may be a complex of human TBK1 and human IKKβ. TBK1 and IKKβ are known to mediate the phosphorylation of threonine at position 749 in human STAT1 (see, e.g., Non Patent Literature 1). The amino acid sequence of human TBK1 may be, for example, the amino acid sequence of NCBI accession No. NP_037386.1 (SEQ ID NO: 3). The nucleotide sequence encoding the human TBK1 may be, for example, the nucleotide sequence of NCBI accession No. NM_013254.4 (SEQ ID NO: 4). The amino acid sequence of human IKKβ may be, for example, the amino acid sequence of NCBI accession No. NP_001547.1 (SEQ ID NO: 5). The nucleotide sequence encoding the human IKKβ may be, for example, the nucleotide sequence of NCBI accession No. NM_001556.3 (SEQ ID NO: 6).

A compound capable of inhibiting the expression or function of human TBK1 and a compound capable of inhibiting the expression or function of human IKKβ may include, for example, but are not limited to, proteins such as anti-human TBK1 antibodies and anti-human IKKβ antibodies; polypeptides that target human TBK1 or human IKKβ; nucleic acid oligos that target human TBK1 or human IKKβ; low-molecular-weight compounds that target human TBK1 or human IKKβ; etc. Preferred examples include proteins, and more preferred examples include antibodies (anti-human TBK1 antibodies or anti-human IKKβ antibodies). Other preferred examples include nucleic acid oligos, and more preferably include siRNAs. Other preferred examples include polypeptides, and more preferably include cyclic polypeptides.

Inhibition of expression of human TBK1 or human IKKβ can be achieved by, for example, utilizing RNA interference effect on each gene expression. RNA interference is an approach reported to silence gene expression utilizing RNA (Genes and Development, 16, 948-958 (2002)). In this phenomenon, a double-stranded RNA having a sequence that is the same as or similar to a target gene is introduced into cells to suppress the expression of both of the introduced exogenous gene and the endogenous target gene. In particular, an antisense nucleic acid or an siRNA that exhibits RNA interference effect on the expression of the human TBK1 gene can be used to inhibit the expression of the human TBK1 gene. Also, an antisense nucleic acid or an siRNA that exhibits RNA interference effect on the expression of the human IKKβ gene can be used to inhibit the expression of the human IKKβ gene.

The term "nucleic acid oligo" refers to a nucleic acid oligomer that controls the function of a target gene or a protein. Nucleic acid oligos may be an oligo of a naturally occurring or non-naturally occurring RNA or DNA. Examples of nucleic acid oligos include, for example, siRNAs, shRNAs, antisense nucleic acids, decoy nucleic acids, nucleic acid aptamers, ribozymes, etc. Preferred examples include siRNAs or shRNAs. The term "siRNA" refers to a short double-stranded RNA with a length that does not induce toxicity in cells. The length of siRNAs may be, for example, from 15 bp to 49 bp, and a suitable length is from 15 bp to 35 bp, and more a suitable length is from 21 bp to 30 bp. The term "shRNA" refers to a single-strand RNA that gains hairpin structure to from a double-strand structure.

siRNA and shRNA used herein are not necessarily completely identical to a target gene, but have at least 70% or more, preferably 80% or more, more preferably 90% or more, or most preferably 95% or more sequence identity to the target gene. The double-stranded RNA region formed by RNA pairing in the siRNA and shRNA may include complete pairing as well as mispairing due to mismatch (with non-complementary nucleotides) or bulge (the lack of a corresponding nucleotide on one strand), etc.

The term "antisense nucleic acid" refers to an antisense nucleic acid complementary to a transcript of DNA encoding a target protein. Antisense nucleic acids can suppress the expression of a target gene through multiple effects, including the following: degradation by RNase activity following recognition of a double-stranded RNA; inhibition of transcription initiation by formation of a triple-stranded nucleic acid; suppression of transcription by formation of a hybrid with an open-loop structure locally generated by RNA polymerase; inhibition of transcription by formation of a hybrid with RNA undergoing synthesis; suppression of splicing by formation of a hybrid with the junction between an exon and an intron; suppression of splicing by formation of a hybrid with a spliceosome assembly site; suppression of translocation of mRNA from the nucleus to the cytoplasm by formation of a hybrid with the mRNA; suppression of translation by formation of a hybrid with a translation initiation factor binding site; prevention of extension of a peptide chain by formation of a hybrid with a translated region or polysome binding site of mRNA; suppression of gene expression by formation of a hybrid with an interacting site between a nucleic acid and a protein; etc. Among these, the antisense nucleic acid as used herein suppresses the expression of a target gene by inhibiting the process of transcription, splicing or translation.

The antisense sequence used in the present invention may be capable of suppressing the expression of a target gene through any of the effects as described above. In one aspect, an antisense sequence complementary to an untranslated region near the 5' end of a target mRNA may be designed to effectively inhibit gene translation. Alternatively, a complementary sequence to a coding region or an untranslated region at the 3' end can also be used. As described, not only a complementary sequence to a translated region of a gene but also a complementary sequence to an untranslated region of a gene can be used. The antisense nucleic acid used in the present invention thus includes a nucleic acid containing an antisense sequence to a translated region of a gene, as well as a nucleic acid containing an antisense sequence to an untranslated region of a gene. The antisense nucleic acid preferably has 90% or more, or most preferably 95% or more complementarity to a transcript of a target gene. The length of an antisense RNA containing an antisense sequence used to effectively inhibit the expression of a target gene is not specifically limited.

The term "ribozyme" refers to, for example, an RNA molecule that cleaves a target mRNA, or an RNA molecule that inhibits translation of a target protein. Ribozymes can be designed from a gene sequence encoding a target protein. For example, hammerhead ribozymes can be constructed according to the method described in FEBS Letters, 228, 228-230 (1988). The ribozymes herein may include any types of ribozymes that cleave the mRNA of a target protein to inhibit translation of the target protein, including hammerhead ribozymes as well as other ribozymes, such as hairpin ribozymes or delta ribozymes.

The term "decoy" means a "lure" in English, and refers to a molecule that has a structure resembling that of a molecule to which a given substance should bind or on which a given substance should act. The term "decoy nucleic acid" refers to a short nucleic acid molecule containing a double-stranded region that binds to a target transcription factor to inhibit the biological function of the transcription factor. Typically, decoy nucleic acids are a double-stranded oligonucleotide that has the same nucleotide sequence as that of the binding region of a transcription factor on genomic DNA. Decoy nucleic acids may further contain a non-complementary single-stranded region, in addition to the double-stranded region formed between complementary strands. Decoy nucleic acids can be administered to reduce the activity of a target transcription factor.

The term "nucleic acid aptamer" refers to an isolated or purified nucleic acid that binds to a target with high specificity and high affinity via a non-Watson-Crick base pair interaction. Aptamers have a three-dimensional structure suitable for formation of specific binding to a target. Aptamer binding differs from the conventional nucleic acid binding in that aptamer binding does not rely on the primary nucleotide sequence of a target, but relies on the specific secondary or tertiary structure of the target. Typical aptamers have a size of 5 to 15 kDa (15 to 45 nucleotides), and can distinguish closely related targets and binds to their own target with an affinity in the nanomolar to sub-nanomolar range (for example, aptamers do not bind to other proteins in the same gene family or derived from the same functional family). Some of aptamers can specifically bind to a selected target to inhibit or activate the function of the target.

The term "cyclic polypeptide" refers to a polypeptide containing a cyclic structure formed of four or more amino acids and/or amino acid analogs. Cyclic polypeptides may have a linear portion in addition to a cyclic portion. The binding mode of a cyclization site is not specifically limited and may be a bond other than an amide bond or an ester bond. The binding mode of a cyclization site is preferably exemplified by, for example, covalent bonds, such as amide bonds, carbon-carbon bonds, disulfide bonds, ester bonds, thioester bonds, thioether bonds, lactam bonds, bonds via an azoline skeleton, bonds via a triazole structure, and bonds via a fluorophore structure. The functional groups used for cyclization, such as carboxy groups and amino groups, may be located on the main chain or on the side chain, and the location of the functional groups is not specifically limited as long as it is located at a cyclizable position. The term "binding mode of a cyclization site" as used herein refers to the binding mode of the cyclization site formed by cyclization reaction. The amino acid analogs herein may be exemplified by, for example, hydroxycarboxylic acid (hydroxy acid).

The molecular weight of the cyclic polypeptide may be 500 to 4,000, 500 to 3,000, or 500 to 2,000. The cyclic polypeptide may contain at least one selected from the group consisting of naturally occurring amino acids, non-naturally occurring amino acids, and amino acid analogs. The ratio of these amino acids contained in the cyclic polypeptide is not specifically limited. The total number of the amino acids and amino acid analogs contained in the cyclic polypeptide of the present invention may be 4 to 20, 4 to 15, 6 to 15, 8 to 15, 9 to 13, or 10 to 13, or may be 5 to 15, 7 to 12, or 9 to 11. When the cyclic polypeptide has a linear portion, the number of the amino acids and/or amino acid analogs contained in the linear portion may be 1 to 8, 1 to 5, or 1 to 3.

The cyclic polypeptide can be produced by any method. For example, the cyclic polypeptide may be identified from cyclic polypeptide libraries using a known method, or may be produced by chemical synthesis methods, such as liquid-phase synthesis methods or solid-phase synthesis methods using Fmoc or Boc.

The polypeptide in the present invention may be modified to enhance the ability to be internalized into cells. Such a modification is not specifically limited and a known method can be used for modification, and the polypeptide may be modified by, for example, attaching a cell membrane-penetrating peptide. The cell membrane-penetrating peptide may be a known sequence, and examples include a Tat peptide derived from HIV Tat protein (Brooks, H. et al. Advanced Drug Delivery Reviews, Vol 57, Issue 4, 2005, p.559-577) or a polyarginine of 6 to 12 arginine residues (Nakase, I. et al. Advanced Drug Delivery Reviews, Vol 60, 2008, p.598-607). It has also been reported that attaching a fatty acid or a stilbene derivative allows the peptide to access the cytoplasmic side (Covic, L. et al. (2002) Nat Med. 8: 1161; Endres, P. J. et al. (2006) Molecular Imaging 4: 485; and Goubaeva, F. et al., J. Biol. Chem. 278: 19634). By using such a method, the polypeptide can be translocated into cells.

### (2) Compound capable of promoting phosphorylation of threonine at position 749 in human STAT1

The compound capable of promoting phosphorylation of threonine 749 in human STAT 1 may be a compound capable of activating a protein kinase that phosphorylates threonine 749 in human STAT1, or a compound capable of promoting binding between human STAT1 and a protein kinase that phosphorylates threonine 749 in human STAT1. The compound capable of activating a protein kinase that phosphorylates threonine 749 in human STAT1 may be a compound capable of promoting the expression of the protein kinase, or a compound capable of enhancing a function of the protein kinase. The compound capable of promoting the expression of a protein kinase that phosphorylates threonine 749 in human STAT1 may be, for example, a nucleic acid molecule encoding the protein kinase, etc. The compound capable of promoting a function of a protein kinase that phosphorylates threonine 749 in human STAT1 may be, for example, a bispecific antibody capable of binding to both of the protein kinase and its substrate human STAT1, or a bispecific antibody capable of binding to both of human TBK1 and human IKKβ, etc. The compound capable of promoting binding between human STAT1 and a protein kinase that phosphorylates threonine 749 in human STAT1 may be a bispecific antibody capable of binding to both of the protein kinase and its substrate human STAT1, etc.

### (3) Compound capable of inhibiting human STAT1

The compound capable of inhibiting human STAT1 may be a compound capable of inhibiting a function of human STAT1, or a compound capable of inhibiting the expression of human STAT1. The compound capable of inhibiting a function or expression of human STAT1 may include, but is not limited to, for example, proteins such as anti-human STAT1 antibodies; polypeptides that target human STAT1; nucleic acid oligos that target human STAT1; low-molecular-weight compounds that target human STAT1; etc. Preferred examples include proteins, and more preferred examples include antibodies (anti-human STAT1 antibodies). Other preferred examples include nucleic acid oligos, and more preferably include siRNAs. Other preferred examples include polypeptides, and more preferably include cyclic polypeptides. The details of each compound are as described in the above section (1) and are omitted herein.

According to the therapeutic agent for an inflammatory disease of the present invention, the compound capable of suppressing phosphorylation of threonine 749 in human STAT1 or the compound capable of inhibiting human STAT1 can be used as an active ingredient of the therapeutic agent for sepsis and/or septic shock. The compound capable of promoting phosphorylation of threonine at position 749 in human STAT1 can be used as an active ingredient of the therapeutic agent for colitis. The compound capable of inhibiting human STAT1 can be used as an active ingredient of the therapeutic agent for systemic lupus erythematosus.

The therapeutic agent for an inflammatory disease of the present invention may contain pharmaceutically acceptable ingredients, such as preservatives, stabilizers, etc. The pharmaceutically acceptable ingredients may be an ingredient that has therapeutic effect on inflammatory diseases on its own, or an ingredient that has no therapeutic effect on inflammatory diseases. Thus, the term "pharmaceutically acceptable" refers to any pharmaceutically acceptable ingredient that can be administered together with the therapeutic agent for an inflammatory disease of the present invention. The pharmaceutically acceptable ingredients may also be an ingredient that has no therapeutic effect but exhibits synergistic effect or additive stabilization effect in conjunction with the active ingredient. Such pharmaceutically acceptable ingredients may include, for example, sterilized water, physiological saline, stabilizers, excipients, buffering agents, antiseptics, surfactants, chelating agents (such as EDTA), binders, etc.

The surfactants may be nonionic surfactants. Typical examples of the nonionic surfactants may include those with an HLB of 6 to 18, including sorbitan fatty acid esters, such as sorbitan monocaprylate, sorbitan monolaurate, or sorbitan monopalmitate; and glycerol fatty acid esters, such as glycerol monocaprylate, glycerol monomyristate, or glycerol monostearate.

The surfactants may also be anionic surfactants. Typical examples of the anionic surfactants include alkyl sulfates having an alkyl group of 10 to 18 carbon atoms, such as sodium cetyl sulfate, sodium lauryl sulfate, and sodium oleyl sulfate; polyoxyethylene alkyl ether sulfates having an average addition molar number of ethylene oxide of 2 to 4 and having an alkyl group of 10 to 18 carbon atoms, such as sodium lauryl polyoxyethylene ether sulfate; salts of alkyl sulfosuccinic acid esters having an alkyl group of 8 to 18 carbon atoms, such as sodium lauryl sulfosuccinate; natural surfactants, for example, lecithin and glycerophospholipid; sphingophospholipids such as sphingomyelin; and sucrose fatty acid esters of fatty acids of 12 to 18 carbon atoms.

The therapeutic agent for an inflammatory disease of the present invention may contain a single surfactant or two or more surfactants in combination as exemplified above. The surfactant for use in a formulation of the present invention is preferably a polyoxyethylene sorbitan fatty acid ester such as polysorbate 20, 40, 60, or 80, particularly preferably polysorbate 20 or 80. Polyoxyethylene polyoxypropylene glycol typified by poloxamer (Pluronic F-68 (registered trademark) etc.) is also preferred.

Examples of the buffering agents may include phosphate buffer solutions, citrate buffer solutions, acetate buffer solutions, malate buffer solutions, tartrate buffer solutions, succinate buffer solutions, lactate buffer solutions, potassium phosphate buffer solutions, gluconate buffer solutions, caprylate buffer solutions, deoxycholate buffer solutions, salicylate buffer solutions, triethanolamine buffer solutions, fumarate buffer solutions, other organic acid buffer solutions, carbonate buffer solutions, Tris buffer solutions, histidine buffer solutions, and imidazole buffer solutions.

A solution formulation may be prepared by dissolution in an aqueous buffer solution known in the field of solution formulations. The concentration of the buffer solution is generally 1 to 500 mM, preferably 5 to 100 mM, more preferably 10 to 20 mM.

The therapeutic agent for an inflammatory disease of the present invention may also contain other low-molecular-weight polypeptides; proteins, such as serum albumin, gelatin, or immunoglobulins; amino acids; saccharides and carbohydrates, such as polysaccharides and monosaccharides; and sugar alcohols. Examples of the saccharides and carbohydrates, such as polysaccharides or monosaccharides, may include dextran, glucose, fructose, lactose, xylose, mannose, maltose, sucrose, trehalose, and raffinose. Examples of the sugar alcohols may include mannitol, sorbitol, and inositol.

For preparation of an aqueous solution for injection, the therapeutic agent may contain, for example, physiological saline, or an isotonic solution containing glucose or other auxiliary agents, such as D-sorbitol, D-mannose, D-mannitol, or sodium chloride. The aqueous solution for injection may further contain an appropriate solubilizing agent, including, for example, alcohols (e.g., ethanol etc.), polyalcohols (e.g., propylene glycol, PEG, etc.), and nonionic surfactants (e.g., polysorbate 80, HCO-50, etc.). The aqueous solution for injection may further contain, if desired, a diluent, a solubilizer, a pH adjusting agent, a soothing agent, a sulfur-containing reducing agent, an antioxidant, etc.

If necessary, the therapeutic agent may be encapsulated in microcapsules (microcapsules made of hydroxymethylcellulose, gelatin, poly[methyl methacrylate], etc.), or made into a colloid drug delivery system (liposomes, albumin microspheres, microemulsions, nanoparticles, nanocapsules, etc.) (see e.g., "Remington's Pharmaceutical Science 16th edition" Oslo Ed., 1980). Methods for formulating an agent into a sustained-release agent are also known in the art, and may be applied to the present invention (Langer et al., J. Biomed. Mater. Res. 1981, 15: 167-277; Langer, Chem. Tech. 1982, 12: 98-105; U.S. Pat. No. 3,773,919; European Patent Publication No. EP 58,481; Sidman et al., Biopolymers 1983, 22: 547-556; and EP 133,988).

The pharmaceutically acceptable carriers used are selected as appropriate or in combination from those described above according to a dosage form, but the pharmaceutically acceptable carriers are not limited to those described above.

When the active ingredient of the therapeutic agent for an inflammatory disease of the present invention is used as a medicament for humans or other animals, the active ingredient can be directly administered to the patient or the patient animal, or the active ingredient can be formulated into a pharmaceutical formulation by known pharmaceutical methods and then administered to the patient or the patient animal. When the active ingredient is formulated into a pharmaceutical formulation, the pharmaceutically acceptable ingredients as described above can be added to the formulation.

The therapeutic agent for an inflammatory disease of the present invention can be administered in the form of a medicament, and can be administered systemically or locally through an oral or parenteral route. For example, intravenous injection, such as drip infusion, intramuscular injection, intraperitoneal injection, subcutaneous injection, a suppository, an enema, or an oral enteric-coated agent can be selected. The mode of administration can be selected as appropriate for the age and symptoms of the patient. The effective dosage is selected from the range of 0.001 mg to 100 mg per kg body weight for each dose. Alternatively, a dose of 0.1 to 1000 mg per patient, preferably 0.1 to 50 mg per patient, can be selected. Specifically, 0.1 mg to 40 mg, preferably 1 to 20 mg, per kg body weight is administered for a month (4 weeks) in a single dose or several divided doses, for example, at a dosing schedule such as twice a week, once a week, once in two weeks, or once in four weeks, by intravenous injection, such as drip infusion, or subcutaneous injection, etc. The dosing schedule may be adjusted in such manner that the dosing intervals are extended from twice a week or once a week to once in two weeks, once in three weeks, or once in four weeks while the conditions of the patient after administration and the trends in blood test values are monitored.

### Screening method

The present invention provides a method for screening for a candidate compound serving as an active ingredient of a therapeutic agent for sepsis and/or septic shock; a method for screening for a candidate compound serving as an active ingredient of a therapeutic agent for colitis; and a method for screening for a candidate compound serving as an active ingredient of a therapeutic agent for systemic lupus erythematosus. The "test compound" to be subjected to the screening methods of the present invention is not specifically limited, and may include, for example, single substances, such as naturally occurring compounds, organic compounds, inorganic compounds, nucleic acid oligos, proteins, polypeptides, or cyclic polypeptides; expression products from compound libraries, nucleic acid oligo libraries, polypeptide libraries, or gene libraries; cell extracts, cell culture supernatants, products of fermentation microorganisms, extracts from marine organisms, plant extracts, extracts from prokaryotic cells, extracts from eukaryotic single cells, and extracts from animal cells. If necessary, such a test compound can be labeled as appropriate. Labeling may be, for example, radiolabeling, fluorescent labeling, etc. The test compound may also include a mixture of different types of test compounds as exemplified above.

### 1. Screening method for candidate compound serving as active ingredient of therapeutic agent for sepsis and/or septic shock

The present invention provides a method for screening for a candidate compound serving as an active ingredient of a therapeutic agent for sepsis and/or septic shock (hereafter referred to as the "screening method for the therapeutic agent for sepsis of the present invention").

In a first embodiment of the screening method for the therapeutic agent for sepsis of the present invention, the method includes selecting a compound capable of inhibiting a protein kinase that phosphorylates threonine 749 in human STAT1 as a compound capable of suppressing phosphorylation of threonine 749 in human STAT1. The particular steps of the method are not specifically limited, and the method may be, for example, a screening method including the following steps (a) and (b):
(a) measuring phosphorylation of human STAT1 by a protein kinase that phosphorylates threonine 749 in human STAT1 in the presence or absence of a test compound, and
(b) selecting the test compound as the candidate compound when the phosphorylation of human STAT1 is suppressed in the presence of the test compound as compared to that in the absence of the test compound.

In step (a), a suitable protein kinase may be human TBK1 and/or human IKKβ. Human STAT1, human TBK1, and human IKKβ used in step (a) can be prepared as a recombinant protein based on the sequence information as described above or the sequence information recorded in known databases (such as NCBI) using known genetic modification technology or recombinant protein expression technology. Commercially available recombinant human STAT1, recombinant human TBK1, and recombinant human IKKβ may also be used. The same applies to other embodiments described later.

The measurement method of phosphorylation of threonine 749 in human STAT1 in step (a) is not specifically limited, and any method appropriately selected from known measurement methods can be used. For example, recombinant human STAT1 as a substrate, recombinant human TBK1 and/or recombinant human IKKβ as protein kinases, and ATP are added to a buffer solution suitable for phosphoryl transfer reaction to initiate the reaction. When radiolabeled ATP (e.g., ³²P-γ-ATP) is used, liquid scintillation counting, autoradiography, phosphoimaging, or the like can be used for the measurement. When radiolabeled ATP is not used, the measurement may be performed by a known immunochemical measurement method (e.g., ELISA, Western blotting, etc.) using an antibody that specifically binds to human STAT1 phosphorylated at threonine 749. Specific methods that can be used are described in, for example, Wen et al., Cell (1995) 82: 241-250, and Ng et al., Proc Natl Acad Sci USA (2011) 108: 21170-21175.

Such an antibody that specifically binds to human STAT1 phosphorylated at threonine 749 is preferably the antibodies of the present invention (described later). For example, the rabbit monoclonal antibodies established by the inventors in the Examples may be used.

In step (b), the test compound is selected as the candidate compound when the phosphorylation of human STAT1 is suppressed in the presence of the test compound as compared to that in the absence of the test compound. The selected compound includes a compound capable of inhibiting a protein kinase that phosphorylates threonine 749 in human STAT1. This compound is capable of suppressing phosphorylation of threonine 749 in human STAT1, thereby potentially exhibiting therapeutic effect on sepsis and/or septic shock.

In a second embodiment of the screening method for the therapeutic agent for sepsis of the present invention, the method includes selecting a compound capable of inhibiting binding between human STAT1 and a protein kinase that phosphorylates threonine 749 in human STAT1 as a compound capable of suppressing phosphorylation of threonine 749 in human STAT1. The particular steps of the method are not specifically limited, and the method may be, for example, a screening method including the following steps (a) and (b):
(a) measuring binding between human STAT1 and a protein kinase that phosphorylates threonine 749 in human STAT1 in the presence or absence of a test compound, and
(b) selecting the test compound as the candidate compound when the binding between human STAT1 and the protein kinase is suppressed in the presence of the test compound as compared to that in the absence of the test compound.

The measurement method of the binding between human STAT1 and the protein kinase in step (a) is not specifically limited, and any method appropriately selected from known measurement methods can be used. The measurement methods may include, for example, ELISA, fluorescence polarization, immunostaining, mass spectroscopy, immunoprecipitation, surface plasmon resonance (SPR), etc. When ELISA is used, one of recombinant human STAT1 and recombinant protein kinase protein (human TBK1 or human IKKβ) is immobilized on a substrate, and the other is added together with a test compound to carry out reaction. Then, the binding level of human STAT1 to the protein kinase can be detected using appropriate primary and secondary antibodies.

In step (b), the test compound is selected as the candidate compound when the binding between human STAT1 and the protein kinase is suppressed in the presence of the test compound as compared to that in the absence of the test compound. The selected compound includes a compound capable of inhibiting binding between human STAT1 and a protein kinase that phosphorylates threonine 749 in human STAT1. This compound is capable of suppressing phosphorylation of threonine 749 in human STAT1, thereby potentially exhibiting therapeutic effect on sepsis and/or septic shock.

In a third embodiment of the screening method for the therapeutic agent for sepsis of the present invention, the method includes selecting a compound capable of specifically binding to threonine at position 749 in human STAT1 as a compound capable of suppressing phosphorylation of threonine 749 in human STAT1. The particular steps of the method are not specifically limited, and the method may be, for example, a screening method including the following steps (a) and (b):
(a) contacting a test compound with wild-type human STAT1 and with mutant human STAT1 having replacement of threonine at position 749 by alanine, and measuring an amount of the test compound bound to either STAT1, and
(b) selecting the test compound as the candidate compound when the amount of the test compound bound to the mutant human STAT1 is smaller than that bound to the wild-type human STAT1.

The measurement method of the binding of a test compound to the wild-type human STAT1 or the mutant human STAT1 in step (a) is not specifically limited, and any method appropriately selected from known measurement methods can be used. For example, the binding can be measured by using a labeled test compound (a radiolabeled test compound, a fluorescent labeled test compound, etc.). In an alternative method, the wild-type human STAT1 and the mutant human STAT1 are separately immobilized on a resin, a chip, etc., then a test compound is contacted with the wild-type and the mutant human STAT1, and the binding is measured. In an alternative method, a test compound is immobilized on a resin, a chip, etc., then the wild-type human STAT1 and the mutant human STAT1 are separately contacted with the test compound, and the binding is measured. Alternatively, surface plasmon resonance (SPR) or other methods can also be used.

In step (b), the test compound is selected as the candidate compound when the amount of the test compound bound to the mutant human STAT1 is smaller than that bound to the wild-type human STAT1. The selected compound includes a compound capable of specifically binding to threonine at position 749 in human STAT1. This compound is capable of suppressing phosphorylation of threonine 749 in human STAT1, thereby potentially exhibiting therapeutic effect on sepsis and/or septic shock.

In a fourth embodiment of the screening method for the therapeutic agent for sepsis of the present invention, the method includes using genetically modified human cells in which a gene encoding mutant human STAT1 having replacement of threonine at position 749 by alanine is knocked-in to screen for a compound capable of suppressing phosphorylation of threonine 749 in human STAT1. The particular steps of the method are not specifically limited, and the method may be, for example, a screening method including the following steps (a), (b) and (c):
(a) providing
   (1) human cells expressing wild-type human STAT1, and
   (2) genetically modified human cells generated by knocking-in a gene encoding mutant human STAT1 having replacement of threonine at position 749 by alanine into the cells as defined in (1),
(b) contacting a test compound with both of the cells as defined in (1) and (2), and
(c) selecting the test compound as the candidate compound when the phosphorylation state of the human STAT1 in the cells as defined in (1) is comparable to that in the cells as defined in (2).

In step (a), (1) human cells expressing wild-type human STAT1, and (2) genetically modified human cells generated by knocking-in a gene encoding mutant human STAT1 having replacement of threonine at position 749 by alanine into the cells as defined in (1) are provided. The human cells expressing wild-type human STAT1 used in step (a) are not specifically limited, and may be, for example, human primary cells or human cell lines naturally expressing human STAT1, or genetically engineered human cell lines modified to express human STAT1 by exogenously introducing a gene encoding human STAT1. The genetically modified human cells in which a gene encoding mutant human STAT1 having replacement of threonine at position 749 by alanine is knocked-in can be prepared by introducing T749A mutation into the STAT1 gene in the human cells expressing wild-type human STAT1 using known genome editing technology.

In step (b), a test compound is contacted with both of the cells as defined in (1) and (2). The test compound may be contacted with the cells by any method, and any method appropriately selected from known methods can be used. For example, a test compound can be added to culture medium containing the cells.

The measurement method of the phosphorylation state of the human STAT1 in the cells in contact with the test compound in step (b) is not specifically limited, and any method appropriately selected from known measurement methods can be used. For example, the phosphorylation state of the human STAT1 in the cells can be measured by extracting protein from the cells by a known method, and performing a known immunochemical measurement method (e.g., ELISA, Western blotting, etc.), peptide-mapping, etc. using an antibody capable of specifically binding to human STAT1 phosphorylated at threonine 749.

In step (c), the test compound is selected as the candidate compound when the phosphorylation state of the human STAT1 in the human cells expressing wild-type human STAT1 as defined in (1) is comparable to that in the genetically modified human cells in which a gene encoding mutant human STAT1 having replacement of threonine at position 749 by alanine is knocked-in as defined in (2). The selected compound is capable of suppressing phosphorylation of threonine at position 749 in human STAT1, thereby potentially exhibiting therapeutic effect on sepsis and/or septic shock.

In a fifth embodiment of the screening method for the therapeutic agent for sepsis of the present invention, the method includes selecting a compound capable of inhibiting a function of human STAT1 as a compound capable of inhibiting human STAT1. The particular steps of the method are not specifically limited, and the method may be, for example, a screening method including the following steps (a) and (b):
(a) measuring a function of human STAT1 in the presence or absence of a test compound, and
(b) selecting the test compound as the candidate compound when the function of human STAT1 is inhibited in the presence of the test compound as compared to that in the absence of the test compound.

The measurement method of a function of human STAT1 in step (a) is not specifically limited, and any method appropriately selected from known measurement methods can be used. For example, the function of STAT1 as a transcription factor can be measured using reporter gene assay etc.

In step (b), the test compound is selected as the candidate compound when the function of human STAT1 is inhibited in the presence of the test compound as compared to that in the absence of the test compound. The selected compound is capable of inhibiting human STAT1, thereby potentially exhibiting therapeutic effect on sepsis and/or septic shock.

In a sixth embodiment of the screening method for the therapeutic agent for sepsis of the present invention, the method includes selecting a compound capable of inhibiting expression of human STAT1 as a compound capable of inhibiting human STAT1. The particular steps of the method are not specifically limited, and the method may be, for example, a screening method including the following steps (a) and (b):
(a) measuring expression of human STAT1 in the presence or absence of a test compound, and
(b) selecting the test compound as the candidate compound when the expression of human STAT1 is inhibited in the presence of the test compound as compared to that in the absence of the test compound.

The measurement method of the expression of human STAT1 in step (a) is not specifically limited, and any method appropriately selected from known measurement methods can be used. For example, cells expressing human STAT1 can be cultured in culture medium containing a test compound or in culture medium not containing the test compound, and then the level of human STAT1 mRNA or protein can be measured. The mRNA level of STAT1 can be determined by extracting RNA from cells by a known method and quantifying mRNA by a known mRNA measurement method. Examples of the known mRNA measurement method include Northern blotting, RT-PCR, quantitative RT-PCR, and RNase protection assay. The protein level of STAT1 can be determined by extracting the protein from cells by a known method and quantifying the protein by a known protein measurement method. Examples of the known protein measurement method include Western blotting, EIA, ELISA, RIA, and a method using a protein measurement reagent.

In step (b), the test compound is selected as the candidate compound when the expression of human STAT1 is inhibited in the presence of the test compound as compared to that in the absence of the test compound. The selected compound is capable of inhibiting expression of human STAT1, thereby potentially exhibiting therapeutic effect on sepsis and/or septic shock.

### 2. Screening method for candidate compound serving as active ingredient of therapeutic agent for colitis

The present invention provides a method for screening for a candidate compound serving as an active ingredient of a therapeutic agent for colitis (hereafter referred to as the "screening method for the therapeutic agent for colitis of the present invention"). Colitis may be any inflammatory disease of the large intestine, and may be, for example, inflammatory bowel disease, ulcerative colitis, Crohn disease, collagenous colitis, lymphocytic colitis, ischemic colitis, diversion colitis, Behcet disease, infectious colitis, or indeterminate colitis.

In a first embodiment of the screening method for the therapeutic agent for colitis of the present invention, the method includes selecting a compound capable of promoting a protein kinase that phosphorylates threonine 749 in human STAT1 as a compound capable of promoting phosphorylation of threonine 749 in human STAT1. The particular steps of the method are not specifically limited, and the method may be, for example, a screening method including the following steps (a) and (b):
(a) measuring phosphorylation of human STAT1 by a protein kinase that phosphorylates threonine 749 in human STAT1 in the presence or absence of a test compound, and
(b) selecting the test compound as the candidate compound when the phosphorylation of human STAT1 is promoted in the presence of the test compound as compared to that in the absence of the test compound.

In step (a), a suitable protein kinase may be human TBK1 and/or human IKKβ. In-house generated or commercially available recombinant human STAT1, recombinant human TBK1, and recombinant human IKKβ can be used in step (a).

The measurement method of phosphorylation of threonine 749 in human STAT1 in step (a) is not specifically limited, and any method appropriately selected from known measurement methods can be used. For example, the measurement can be done in the same manner as in the first embodiment of the screening method for the therapeutic agent for sepsis of the present invention.

In step (b), the test compound is selected as the candidate compound when the phosphorylation of human STAT1 is promoted in the presence of the test compound as compared to that in the absence of the test compound. The selected compound includes a compound capable of activating a protein kinase that phosphorylates threonine 749 in human STAT1. This compound is capable of promoting phosphorylation of threonine 749 in human STAT1, thereby potentially exhibiting therapeutic effect on colitis.

In a second embodiment of the screening method for the therapeutic agent for colitis of the present invention, the method includes selecting a compound capable of promoting binding between human STAT1 and a protein kinase that phosphorylates threonine 749 in human STAT1 as a compound capable of promoting phosphorylation of threonine 749 in human STAT1. The particular steps of the method are not specifically limited, and the method may be, for example, a screening method including the following steps (a) and (b):
(a) measuring binding between human STAT1 and a protein kinase that phosphorylates threonine 749 in human STAT1 in the presence or absence of a test compound, and
(b) selecting the test compound as the candidate compound when the binding between human STAT1 and the protein kinase is promoted in the presence of the test compound as compared to that in the absence of the test compound.

The measurement method of the binding between human STAT1 and the protein kinase in step (a) is not specifically limited, and any method appropriately selected from known measurement methods can be used. The measurement can be done in the same manner as in the second embodiment of the screening method for the therapeutic agent for sepsis of the present invention.

In step (b), the test compound is selected as the candidate compound when the binding between human STAT1 and the protein kinase is promoted in the presence of the test compound as compared to that in the absence of the test compound. The selected compound includes a compound capable of promoting the binding between human STAT1 and a protein kinase that phosphorylates threonine 749 in human STAT1. This compound is capable of promoting phosphorylation of threonine 749 in human STAT1, thereby potentially exhibiting therapeutic effect on colitis.

### 3. Screening method for candidate compound serving as active ingredient of therapeutic agent for systemic lupus erythematosus

The present invention provides a method for screening for a candidate compound serving as an active ingredient of a therapeutic agent for systemic lupus erythematosus (hereafter referred to as the "screening method for the therapeutic agent for systemic lupus erythematosus of the present invention").

In a first embodiment of the screening method for the therapeutic agent for systemic lupus erythematosus of the present invention, the method includes selecting a compound capable of inhibiting a function of human STAT1 as a compound capable of inhibiting human STAT1. The particular steps of the method are not specifically limited, and the method may be, for example, a screening method including the following steps (a) and (b):
(a) measuring a function of human STAT1 in the presence or absence of a test compound, and
(b) selecting the test compound as the candidate compound when the function of human STAT1 is inhibited in the presence of the test compound as compared to that in the absence of the test compound.

The measurement method of a function of human STAT1 in step (a) is not specifically limited, and any method appropriately selected from known measurement methods can be used. For example, the measurement can be done in the same manner as in the fifth embodiment of the screening method for the therapeutic agent for sepsis of the present invention.

In step (b), the test compound is selected as the candidate compound when the function of human STAT1 is inhibited in the presence of the test compound as compared to that in the absence of the test compound. The selected compound is capable of inhibiting human STAT1, thereby potentially exhibiting therapeutic effect on systemic lupus erythematosus.

In a second embodiment of the screening method for the therapeutic agent for systemic lupus erythematosus of the present invention, the method includes selecting a compound capable of inhibiting expression of human STAT1 as a compound capable of inhibiting human STAT1. The particular steps of the method are not specifically limited, and the method may be, for example, a screening method including the following steps (a) and (b):
(a) measuring expression of human STAT1 in the presence or absence of a test compound, and
(b) selecting the test compound as the candidate compound when the expression of human STAT1 is inhibited in the presence of the test compound as compared to that in the absence of the test compound.

The measurement method of the expression of human STAT1 in step (a) is not specifically limited, and any method appropriately selected from known measurement methods can be used. For example, the measurement can be done in the same manner as in the sixth embodiment of the screening method for the therapeutic agent for sepsis of the present invention.

In step (b), the test compound is selected as the candidate compound when the expression of human STAT1 is inhibited in the presence of the test compound as compared to that in the absence of the test compound. The selected compound is capable of inhibiting expression of human STAT1, thereby potentially exhibiting therapeutic effect on systemic lupus erythematosus.

### Antibody capable of specifically binding to human STAT1 phosphorylated at threonine at position 749

The present invention provides an antibody capable of specifically binding to human STAT1 phosphorylated at threonine at position 749 (hereafter referred to as the "antibody of the present invention"). The antibody of the present invention may be an antibody that cross-reacts with mouse STAT1 phosphorylated at threonine at position 748. The amino acid sequence of mouse STAT1 may be, for example, the amino acid sequence of NCBI accession No. NP_001192243.1 (SEQ ID NO: 7). The nucleotide sequence encoding the mouse STAT1 may be, for example, the nucleotide sequence of NCBI accession No. NM_001205314.1 (SEQ ID NO: 8). The antibody of the present invention that cross-reacts with human STAT1 phosphorylated at threonine at position 749 and mouse STAT1 phosphorylated at threonine at position 748 may also called herein the "antibody capable of binding to STAT1 phosphorylated at threonine at the second position from the C-terminus" or the "antibody capable of binding to STAT1 phosphorylated at threonine that is closest to the C-terminus."

The term "antibody" herein is used in its widest meaning, and includes various antibody structures that exhibit the desired antigen binding activity, such as monoclonal antibodies, polyclonal antibodies, multi-specific antibodies (e.g., bispecific antibodies), and antibody fragments. An antibody may be a mouse, rabbit, human, humanized or chimeric antibody, or may be derived from other species. An antibody may be of any type (e.g., IgG, IgE, IgM, IgD, or IgA), any class (e.g., IgG1, IgG2, IgG3, IgG4, IgA1, or IgA2), or any subclass of immunoglobulin molecules. The term "antibody fragment" refers to a portion of an antibody, preferably containing a variable domain of an antibody, or an antigen binding region of an antibody. An antibody fragment may include Fab, Fab', F(ab')₂, a Fv fragment, a linear antibody, a single-chain antibody (scFv), sc(Fv)₂, Fab₃, a domain antibody (dAb) (WO 2004/058821, WO 2003/002609), a diabody, a triabody, a tetrabody, a minibody, etc.

The antibody of the present invention has higher binding affinity to STAT1 phosphorylated at the target threonine than to wild-type STAT1. The term "wild-type STAT1" as used herein means non-phosphorylated STAT1 in which none of amino acid residues are phosphorylated. The binding affinity of the antibody to an antigen can be determined by, for example, a known method, such as ELISA.

The antibody of the present invention is preferably a monoclonal antibody. The monoclonal antibody can be prepared by a known method. For example, a fragment of human STAT1 containing phosphorylated threonine 749 or a fragment of mouse STAT1 containing phosphorylated threonine 748 is dissolved in PBS, and an appropriate amount of a commonly used adjuvant (e.g., Freund's complete adjuvant) is added thereto, if desired. The prepared solution is then used as an immunogen to immunize a mammal (e.g., a mouse, a rat, a rabbit, a goat, a horse, etc.). The immunization method is not specifically limited, and may, for example, be performed by subcutaneous or intraperitoneal injection given once or repeatedly several times at appropriate intervals. Immune cells (e.g., splenocytes) are harvested from the immunized mammal and then fused with myeloma cells to produce hybridomas. The monoclonal antibody can be obtained by collecting antibodies from the culture of the hybridomas.

The antibody of the present invention is preferably a rabbit monoclonal antibody. Rabbit antibodies are known to typically have higher affinity and higher specificity to their targets, and rabbits can produce antibodies against many antigens that exhibit poor immunogenicity to mice and other animals. A rabbit monoclonal antibody can be produced as a recombinant rabbit monoclonal antibody by, for example, using the ImmunoSpot Array Assay on a Chip (ISAAC) method (Nat. Med., 15, 1088-1092, 2009, and PLoS One, 7, 1-9, 2012), in which antibody-producing cells are screened, and an antibody gene from the selected antibody-producing cells is cloned and introduced into host cells (see Example 4).

The antibody of the present invention is preferably rabbit monoclonal antibodies produced by antibody-producing cell clone #1 and clone #2 selected in Example 4. The rabbit monoclonal antibodies produced by antibody-producing cell clone #1 and clone #2 have been determined to have high specificity to both of human STAT1 phosphorylated at threonine at position 749 and mouse STAT1 phosphorylated at threonine at position 748, to have cross-reactivity, and to have low binding affinity to non-phosphorylated human and mouse STAT1.

The antibody of the present invention is capable of rapidly and specifically detecting the phosphorylation state at position 749 of human STAT1, and is suitable for use in the screening method of the present invention. The antibody of the present invention is very useful as a study tool on STAT1.

### Genetically modified cells

The present invention also provides a genetically modified human cell in which a gene encoding mutant human STAT1 having replacement of threonine at position 749 by alanine is knocked-in, and a genetically modified mouse cell in which a gene encoding mutant mouse STAT1 having replacement of threonine at position 748 by alanine is knocked-in.

The genetically modified cells of the present invention can be prepared by, for example, using known genome editing technology. For example, the genetically modified mouse cells in which a gene encoding mutant mouse STAT1 having replacement of threonine at position 748 by alanine is knocked-in can be prepared by introducing, into mouse cells, the template oligo DNA described in the section titled "1. Mice" in "Experimental materials and experimental methods" in Examples described later together with crRNA/tracrRNA/Cas9 complex. Similarly, the genetically modified human cells in which a gene encoding mutant human STAT1 having replacement of threonine at position 749 by alanine is knocked-in can be prepared by designing a template oligo DNA for introducing T749A mutation into the human STAT1 gene, and then introducing the template oligo DNA with crRNA/tracrRNA/Cas9 complex into human cells. All the cells isolated from the Stat1T748A mutant mice described in the section titled "1. Mice" in "Experimental materials and experimental methods" in Examples are the genetically modified mouse cells in which a gene encoding mutant mouse STAT1 having replacement of threonine at position 748 by alanine is knocked-in according to the present invention.

The genetically modified cells of the present invention are useful as a tool for evaluating whether a test compound can specifically suppress only the phosphorylation at position 749 of human STAT1, and is suitable for use in the screening method of the present invention (see the fourth embodiment of the screening method for the therapeutic agent for sepsis of the present invention).

### EXAMPLES

The present invention will be described in more detail below with reference to Examples, but the present invention is not limited thereto.

### Experimental materials and experimental methods

### 1. Mice

Stat1T748A mutant mice on a C57BL/6 background were generated by using the CRISPR/Cas9 method. Briefly, a template oligo DNA (5'-ccaaagtccgtgtactgaacctacctacattctgtctcttgcagATGAGCGCTGTATGAtgaattctctggcgacatttttttc ccatctgtgattccttcctgctactgttcctt-3': SEQ ID NO: 9) was designed to introduce T748A mutation (underlined upper-case letters) as well as EcoRI site (underlined lower-case letters) for further genotyping. Several silent mutations were introduced to prevent recurrent cleavages. C57BL/6 zygotes were electroporated with crRNA/tracrRNA/Cas9 complex with the template oligo. After recombination, the ACA sequence encoding a threonine (T) residue in WT Stat1 was replaced by GCT, encoding an alanine (A) residue. Stat1KO on a C57BL/6 background (Kimura et al., J. Exp. Med., 206, 2027-2035, 2009) was backcrossed with Stat1T748A mutant mice for three generations. Wt/Stat1T748A was crossed with Stat1KO/Stat1T748A to generate isogenic littermates. The breeding followed the Mendelian inheritance pattern. Mice were kept and bred in pathogen-free conditions. Age and sex matched littermates were used for the experiments unless otherwise indicated herein. All animal experiments were conducted in accordance with the guidelines of the Animal Care and Use Committee of Osaka University.

### 2. Genotyping and DNA sequencing

Genomic DNA was extracted from the tail using Tail lysis buffer with proteinase K (Nacalai Tesque). DNA was cleaned using Protein Precipitation Solution (Promega), and DNA was precipitated using isopropanol. Then, DNA was cleaned with 70% ethanol and eluted with Tris-EDTA (TE) buffer. The PCR primers as shown below were used for genotyping of Wt/Stat1T748A. The PCR product size is 970 bp.
forward: 5'-ACAGCCACCTAAGACATCTGG-3' (SEQ ID NO: 10)
reverse: 5'-AGCACTTGGCACTGATTACAGAC-3' (SEQ ID NO: 11)

Then, the PCR products were subjected to restriction digestion using EcoRI or AfeI, followed by DNA electrophoresis resulting in different band patterns corresponding to the genotypes. In this study, the AfeI enzyme was used for genotyping as it digests directly within the T748A mutation site (AGCGCT).

For Stat1KO/Stat1T748A genotyping, two different PCR reactions were performed using a common forward primer and different reverse primers corresponding to either Stat1 exon or the neomycin cassette replacing the Stat1 exon (Stat1KO). The primers used are shown below. The size of the PCR product of Stat1T748A is 360 bp, and the size of the PCR product of the Stat1KO is 440 bp.
common forward: 5'-TCTAAGGCAGAGATGGTTAGCATGG-3' (SEQ ID NO: 12)
Stat1 reverse: 5'-CAGTGCCTCTCAACCTTCCTGACAC-3' (SEQ ID NO: 13)
neomycin reverse: 5'-ATTCGCAGGGCATCGCCTTCTAT-3' (SEQ ID NO: 14)

To confirm the Stat1T748A mutation using DNA sequencing, the PCR products were purified using QIAquick PCR Purification Kit (QIAGEN). DNA sequencing primer used was 5'-AGGAGGCCAGACAGTAATAACC-3' (SEQ ID NO: 15).

### 3. Lipopolysaccharide (LPS)-induced septic shock model

Ten- to twelve-week-old male littermate mice were injected intraperitoneally with LPS (L2880, Sigma) dissolved in PBS. For LPS-induced lethality, mice were injected with 12.5 mg/kg body weight. Mice were assessed twice a day for survival. For experimental analysis, mice were injected with a sublethal dose of 7.5 mg/kg body weight of LPS for the indicated time points. At the indicated times after LPS injection, the animals were killed by CO₂ overdose and rapidly dissected. Spleens were isolated and processed for single-cell preparation for the downstream analysis.

### 4. Dextran sulfate sodium (DSS)-induced colitis model

Ten- to twelve-week-old male littermate mice were allowed to free access to a 1.5% (w/v) DSS (36-50 kDa, MP Biomedical) solution as a drinking water for 7 days to induce acute colitis. Changes in the body weight, feces and colonic tissue of mice were analyzed. On day 7 after the start of administration of 1.5% DSS, mice were euthanized and large intestines were isolated. The feces were carefully removed from the entire large intestines, and the large intestines were fixed in 4% PBS-buffered paraformaldehyde. Preparation of histopathological specimens and hematoxylin & eosin (H&E) staining were commissioned to Kyoto Institute of Nutrition & Pathology, Inc. (Kyoto, Japan). The images were photographed using BZ-X710 microscope (KEYENCE). The severity of colitis was assessed by a blind test as previously described (Sato et al., Sci. Rep., 5, 2015).

### 5. Pristane-induced lupus model

Seven- to eight-week-old female littermate mice were intraperitoneally received 0.5 ml of pristane (Sigma), and monitored for 6 months. The survival, serum anti-dsDNA titers and urinary protein of mice were analyzed. Serum anti-dsDNA antibody titers were determined using LBIS Mouse anti-dsDNA ELISA Kit (FUJIFILM Wako Pure Chemical Corporation). Albuminuria was determined as the amount of albumin (µg) per mg of creatinine in midstream urine, and measured using Mouse Urinary Albumin Quantitative ELISA Kit (Ethos Bioscience) and Urinary Creatinine Quantitation Kit Creatinine Companion (Ethos Bioscience) according to the manufacturer's protocols. For assessment of glomerulonephritis, kidneys were isolated from pristane administration mice or non-pristane administration mice, and fixed in 4% PBS-buffered paraformaldehyde for 1 hour. Then, buffer solution was exchanged to 10% PBS-buffered saccharide and then to 20% PBS-buffered saccharide. Preparation of histopathological specimens and hematoxylin & eosin (H&E) staining were commissioned to Kyoto Institute of Nutrition & Pathology, Inc. (Kyoto, Japan). The images were photographed using BZ-X710 microscope (KEYENCE).

### 6. Plasmid construction and mutagenesis

Recombinant overexpressing vectors encoding human Flag-STAT1WT, human Flag-STAT1T749A and human HA-IKKβ were used as previously described (Metwally, H et al., SCIENCE SIGNALING, 2020 Mar 24;13(624): eaay0574). Mouse Flag-Stat1WT and 3xFlag-Ikkβ constructs were prepared using In-Fusion HD cloning kit (Clontech) according to manufacturer's protocol. Stat1 (ENSMUST00000070968.14) and Ikkb (ENSMUST00000033939.13) constructs were prepared by PCR-based amplification from cDNA of mouse macrophages. Mouse Flag-Stat1T748A mutant construct was generated using KOD-Plus-Mutagenesis Kit (TOYOBO) according to manufacturer's protocol. The following primers were used:
Mouse Stat1 F: 5'-ATGTCACAGTGGTTCGAGCTTC-3' (SEQ ID NO: 16)
Mouse Stat1 R: 5'-TTATACTGTGCTCATCACTGTC-3' (SEQ ID NO: 17)
Mouse Ikkβ F: 5'-ATGAGCTGTCACCGTCCCTC-3' (SEQ ID NO: 18), and
Mouse Ikkβ R: 5'-TCAGTCACAGGCCTGCTCCAG-3' (SEQ ID NO: 19).

### 7. Western blot and immunoprecipitation

Whole-cell lysates were prepared using Pierce IP Lysis Buffer (Thermo Fisher). Protein concentration was quantified using DC protein assay (BioRad, 500-0116). Lysates were boiled in SDS sample buffer (Nacalai Tesque) at 96°C for 5 minutes, and separated by 5-20% SDS-PAGE gels (Nacalai Tesque). Precision Plus Protein 2 Color Standard (BioRad) was used as a molecular marker. Proteins were then transferred onto a 0.45 µm polyvinylidene difluoride (PVDF) membrane (GE Healthcare) by wet transfer system using Criterion Blotter (BioRad). Then, the membrane was blocked in 5% w/v skim milk suspended in 1x Tris-Buffered Saline (TBST) containing 0.1% Tween-20 at room temperature for 2 hours. The membrane was washed with TBST, and incubated with primary antibodies in Can Get Signal Immunereaction Enhancer (TOYOBO, NKB-101T) Solution 1 (1/1000 dilution) at 4°C overnight. Then, the membrane was washed 3 times with TBST, and incubated with Amersham ECL anti-rabbit or anti-mouse IgG horseradish peroxidase (HRP) conjugated (GE Healthcare) in Can Get Signal Immunereaction Enhancer Solution 2 (1/10000 dilution) at room temperature for 1 hour. For detection of pThr Stat1, primary antibodies were diluted in 3% BSA in TBST, and secondary antibodies diluted in 5% skim milk were used. After incubation with the secondary antibodies, the membrane was washed 3 times with TBST. Blots were developed using ECL Prime Western Blotting Detection Reagent (Cytvia), and images were taken by Image Quant LAS800 (GE Healthcare).

For detection of biotinylated pThr Stat1, whole-cell lysates were treated with streptavidin magnetic beads (NEB) prior to the protein measurement step.

For immunoprecipitation, whole-cell lysates were pretreated, and proteins in the lysates were immunoprecipitated using the indicated antibodies at 4°C overnight. Then, Dynabeads Protein G for Immunoprecipitation (Thermo Fisher) were added to collect immunocomplexes at 4°C for 2 hours. Then, the antibody-bound beads were washed 4 times with lysis buffer, and eluted using Pierce IgG Elution Buffer (Thermo Fisher). Eluates were boiled at 96°C for 5 minutes in SDS sample buffer, and subjected to Western blot analysis.

The antibodies used for Western blotting and immunoprecipitation are shown below. These antibodies were purchased from Cell Signaling and Technology (CST): Stat1 (D1K9Y) Rabbit mAb (#14994), IKKβ (D30C6) Rabbit mAb (#8943), Stat2 (D9J7L) Rabbit mAb (#72604), Stat3 (D3Z2G) Rabbit mAb (#12640), Stat5 (D2O6Y) Rabbit mAb (#94205), IκBα (44D4) Rabbit mAb (#4812), p44/42 MAPK (Erk1/2) (137F5) Rabbit mAb (#4695), Phospho-Stat1 (Tyr701) (58D6) Rabbit mAb (#9167), Phospho-Stat1 (Ser727) (D3B7) Rabbit mAb (#8826), β-Actin (8H10D10) Mouse mAb (HRP Conjugate) (#12262), Anti-rabbit IgG HRP-linked Antibody (#7074), and Streptavidin-HRP (#3999).

### 8. Statistical analysis

Survival was analyzed using Kaplan-Meier curves and log-rank (Mantel-Cox) test. One-way ANOVA with post-hoc Tukey's test, unpaired two-tailed Student t-test with Welch's correction, Mann-Whitney U test, and Kruskal-Wallis with post-hoc Dunn test were used to determine p values. P value < 0.05 was considered statistically significant. All statistical analyses were performed using GraphPad Prism software.

### Example 1: Evaluation of Stat1 phosphorylated at threonine 748 in LPS-induced septic shock

### 1-1 Survival after LPS challenge

### (1) Evaluation of survival of wild-type mice (Wt/Wt), heterozygous mice (Wt/Stat1T748A) and mutant mice (Stat1T748A/Stat1T748A)

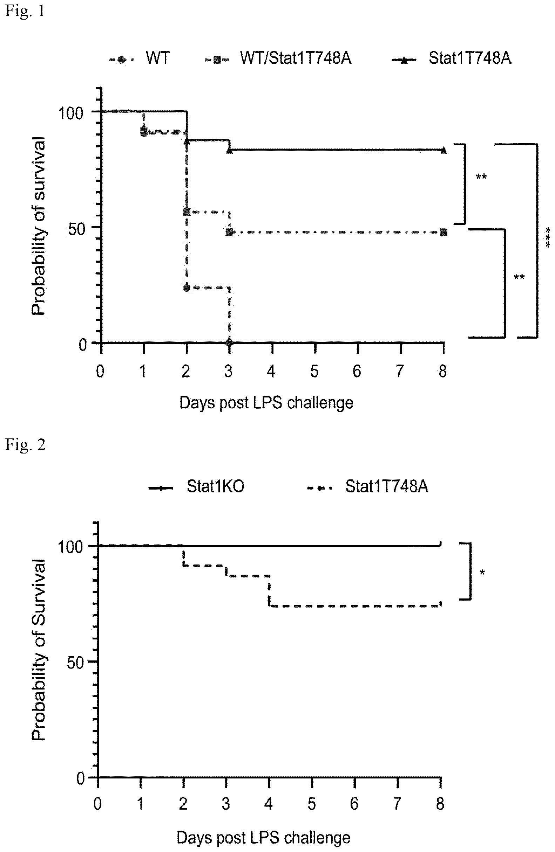

Wt/Wt (hereafter referred to as "Wt"), Wt/Stat1T748A (hereafter referred to as "heterozygous"), and Stat1T748A/Stat1T748A (hereafter referred to as "Stat1T748A") littermates were generated by mating Wt/Stat1T748A mice. Survival of mice were evaluated following intraperitoneal administration of LPS (12.5 mg/kg). The results are shown in Fig. 1. Stat1T748A showed the highest survival, whereas all Wt littermates were dead on day 3 after LPS administration, and heterozygous exhibited intermediate survival between the groups. The results suggested that phosphorylation of Stat1T748 promotes host inflammatory reaction in septic shock. The data shown in Fig. 1 were collected from four independent experiments each using n = 4-6 mice for each genotype.

### (2) Evaluation of survival of Stat1-deficient mice (Stat1KO/Stat1KO) and mutant mice (Stat1T748A/Stat1T748A)

Stat1KO/Stat1KO (hereafter referred to as "Stat1KO") and Stat1T748A littermates were generated by mating Stat1KO/Stat1T748A mice. Survival of mice were evaluated following intraperitoneal administration of LPS (12.5 mg/kg). The results are shown in Fig. 2. Stat1KO was resistant to LPS-induced lethality. Stat1T748A exhibited high survival, as with that shown in Fig. 1. The data shown in Fig. 2 were collected from four independent experiments each using n = 4-6 mice for each genotype.

### 1-2 Expression of endogenous proteins in splenocytes from Wt, Stat1T748A and Stat1KO

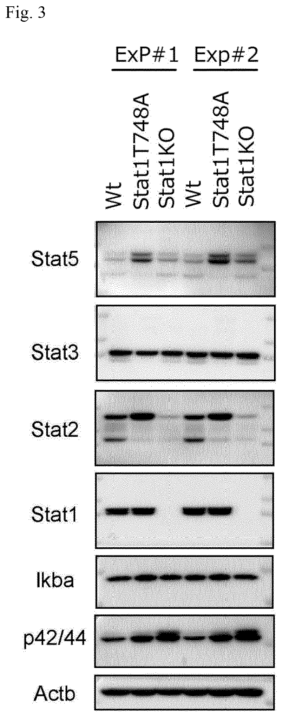

Spleens were isolated from Wt and Stat1T748A mice (littermates generated by mating Wt/Stat1T748A mice) and Stat1KO mice that did not receive LPS. Whole-cell lysates were prepared and separated by SDS-PAGE for Western blotting analysis. The results are shown in Fig. 3. The results confirmed that Stat1T748A has no abnormalities in expression of Stat1 or other STAT proteins.

The overall results revealed that phosphorylation of threonine 748 in Stat1 greatly contributes to inflammatory functionality of Stat1 in LPS-induced septic shock. This suggested that septic shock and/or sepsis can be treated by inhibiting phosphorylation of threonine 749 in human Stat1 corresponding to threonine 748 in mouse Stat1, or inhibiting the function or expression of human Stat1. The results also indicated that a substance capable of inhibiting phosphorylation of threonine 749 in human Stat1 corresponding to threonine 748 in mouse Stat1, or a substance capable of inhibiting the function or expression of human Stat1 can be a candidate substance as a therapeutic drug for septic shock and/or sepsis.

### Example 2: Evaluation of Stat1 phosphorylated at threonine 748 in dextran sulfate sodium (DSS)-induced colitis

### (1) Evaluation of colitis in wild-type mice (Wt) and mutant mice (Stat1T748A)

Wt and Stat1T748A littermates generated by mating Wt/Stat1T748A mice were allowed to free access to a 1.5% (w/v) DSS solution as a drinking water for 7 days to induce acute colitis. The body weight was measured every day prior to DSS administration (Day 0). On day 7 after the start of administration of DSS (Day 7), mice were euthanized and large intestines were isolated, and the colon length was measured. Histopathological specimens of the colon were prepared to evaluate the severity of colitis.

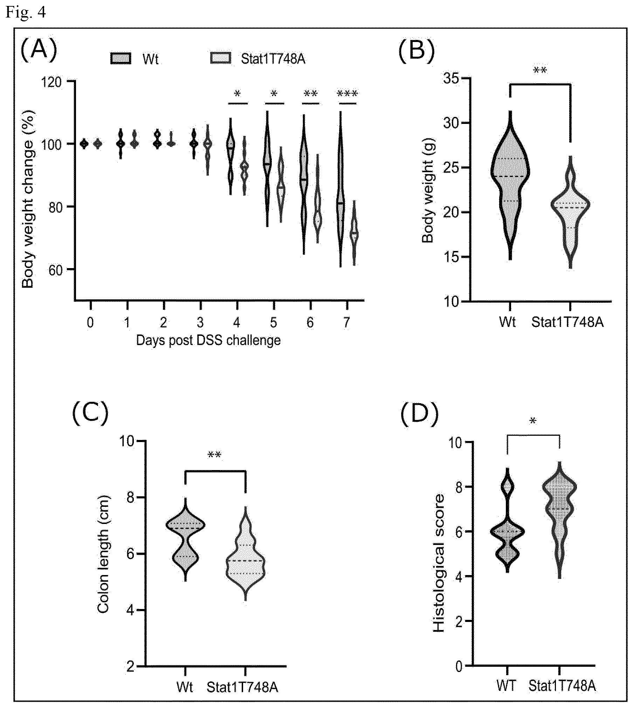

The results are shown in Fig. 4. Panel (A) shows the weight change during the DSS administration period. Panel (B) shows the body weight on day 7. Panel (C) shows the colon length. Panel (D) shows the severity score of colitis in histopathological test of colon. Stat1T748A showed significant decrease in the body weight, significant decrease in the colon length, significant increase in the severity score of colitis, and severe inflammation in the intestines, as compared to Wt. The data shown in Fig. 4 were collected from four independent experiments each using n = 3 mice for each genotype.

### (2) Evaluation of colitis in Stat1-deficient mice (Stat1KO) and mutant mice (Stat1T748A)

Stat1KO and Stat1T748A littermates generated by mating Stat1KO/Stat1T748A mice were allowed to free access to a 1.5% (w/v) DSS solution as a drinking water for 7 days to induce acute colitis. The body weight was measured every day prior to DSS administration (Day 0). On day 7 after the start of administration of DSS (Day 7), mice were euthanized and large intestines were isolated, and the colon length was measured. Histopathological specimens of the colon were prepared to evaluate the severity of colitis.

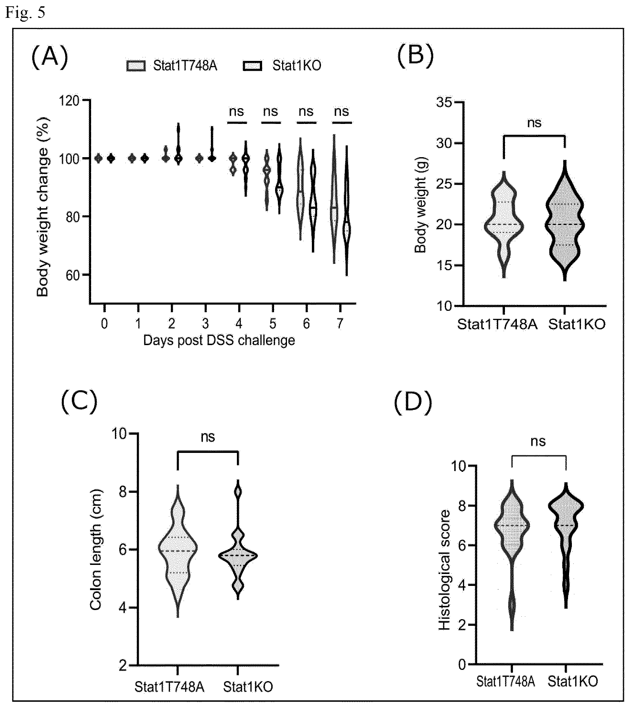

The results are shown in Fig. 5. Panel (A) shows the weight change during the DSS administration period. Panel (B) shows the body weight on day 7. Panel (C) shows the colon length. Panel (D) shows the severity score of colitis in histopathological test of colon. The body weight, colon length, and severity score of colitis of Stat1T748A were comparable to those of Stat1KO. The data shown in Fig. 5 were collected from four independent experiments each using n = 4-5 mice for each genotype.

The overall results revealed that phosphorylation of threonine 748 in Stat1 is important for prevention of advancement of colitis. This suggested that colitis can be treated by promoting phosphorylation of threonine 749 in human Stat1 corresponding to threonine 748 in mouse Stat1. The results also indicated that a substance capable of promoting phosphorylation of threonine 749 in human Stat1 corresponding to threonine 748 in mouse Stat1 can be a candidate substance as a therapeutic drug for colitis.

### Example 3: Evaluation of Stat1 phosphorylated at threonine 748 in pristane-induced lupus

Pristane-induced lupus model mice are considered to serve as a systemic lupus erythematosus (SLE) model since the model mice present symptoms similar to those of human SLE, such as production of IgG autoantibodies against nucleic acid-related antigens or glomerulonephritis.

### (1) Evaluation of lupus conditions in wild-type mice (Wt) and mutant mice (Stat1T748A)

Wt and Stat1T748A littermates generated by mating Wt/Stat1T748A mice were intraperitoneally received 0.5 ml of pristane, and monitored for 6 months. The survival, serum anti-dsDNA titers and urinary protein of mice were analyzed. Kidneys were isolated from pristane administration mice or non-pristane administration mice, and histopathological specimens were prepared to evaluate glomerulonephritis.

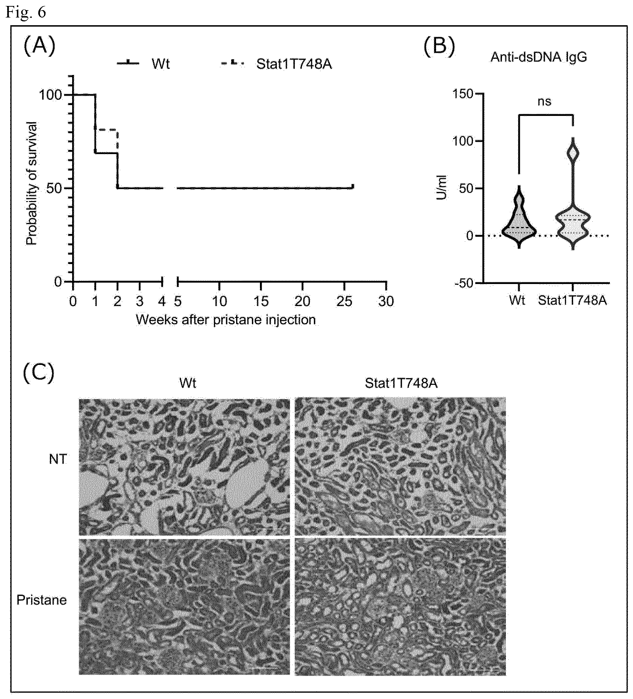

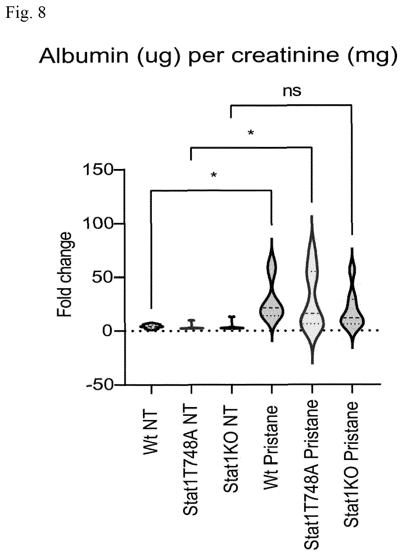

The results are shown in Fig. 6. Panel (A) shows the survival for 6 months after pristane administration. Panel (B) shows the serum anti-dsDNA titers 6 months after pristane administration. Panel (C) shows microphotographs of the hematoxylin and eosin specimens of kidney sections. The results of urinary protein combined with the results from the section (2) below are shown in Fig. 8. The survival of Stat1T748A was comparable to that of Wt, which was about 50%. The serum anti-dsDNA titers, the urinary protein, and the histopathological images of glomerulonephritis were comparable between Stat1T748A and Wt.

### (2) Evaluation of lupus conditions in Stat1-deficient mice (Stat1KO) and mutant mice (Stat1T748A)

Stat1KO and Stat1T748A littermates generated by mating Stat1KO/Stat1T748A mice were intraperitoneally received 0.5 ml of pristane, and monitored for 6 months. The survival, serum anti-dsDNA titers and urinary protein of mice were analyzed. Kidneys were isolated from pristane administration mice or non-pristane administration mice, and histopathological specimens were prepared to evaluate glomerulonephritis.

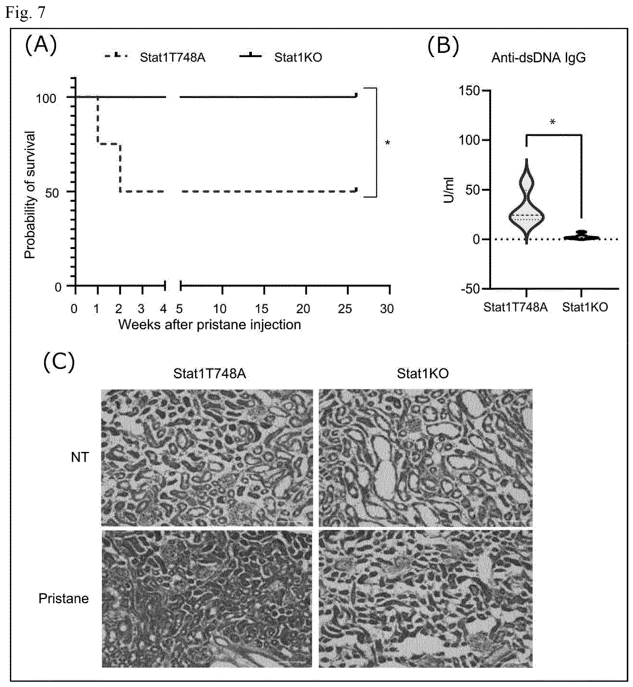

The results are shown in Fig. 7. Panel (A) shows the survival for 6 months after pristane administration. Panel (B) shows the serum anti-dsDNA titers 6 months after pristane administration. Panel (C) shows microphotographs of the hematoxylin and eosin specimens of kidney sections. Stat1KO mice were resistant to pristane-induced lethality and showed lower serum anti-dsDNA titers, as compared to those of Stat1T748A mice. The pathological conditions indicated by the urinary protein and the histopathological images of glomerulonephritis were also milder than those of Stat1T748A.

The overall results revealed that Stat1KO suppresses the pristane-induced conditions of systemic lupus erythematosus, whereas phosphorylation of threonine 748 in Stat1 is not involved in suppression of the conditions of systemic lupus erythematosus. This suggested that systemic lupus erythematosus can be treated by inhibiting the function or expression of human Stat1. The results also indicated that a substance capable of inhibiting the function or expression of human Stat1 can be a candidate substance as a therapeutic drug for systemic lupus erythematosus.

### Example 4: Generation of rabbit monoclonal antibody against mouse STAT1 phosphorylated at threonine at position 748 (hereafter referred to as "pThr748 mStat1") and rabbit monoclonal antibody against human STAT1 phosphorylated at threonine at position 749 (hereafter referred to as "pThr749 hStat1")

pThr748 mStat1 peptide used was a peptide phosphorylated at threonine at position 748 in the C-terminal region (positions 736 to 749 of SEQ ID NO: 7) of mouse Stat1 (RIVGPEFDSMMS(pT)V, SEQ ID NO: 20). Keyhole limpet hemocyanin (KLH) was conjugated to the N-terminus of the peptide via cysteine (C). Twelve- to thirteen-week-old New Zealand White rabbits were immunized subcutaneously with 500 µg of KLH conjugates of pThr748 mStat1 peptide in complete Freund's adjuvant (Millipore). Two, four, and six weeks after primary immunization, the immunization was boosted by subcutaneously inoculating the rabbits with 500 µg of KLH conjugates of pThr748 mStat1 peptide in incomplete Freund's adjuvant (Millipore). One week after the final boost, peripheral blood leukocytes (PBLs) were separated by centrifugation on a Ficoll-Hypaque gradient, and rabbit IgG+ cells were isolated with rabbit IgG-specific antibody-conjugated microbeads (Miltenyi Biotec) and MS columns (Miltenyi Biotec) according to the manufacturer's instructions.

pThr748 mStat1-specific antibody-producing cells and pThr749 hStat1-specific antibody-producing cells were screened using the ISAAC (ImmunoSpot Array Assay on a Chip) method. The microwell array chip and the ISAAC method were followed as reported by Jin et al. (Nat. Med., 15, 1088-1092, 2009) and Ozawa et al. (PLoS One, 7, e52383, 2012). Briefly, the surface of the chip was coated with 10 µg/ml rabbit IgG-specific antibody (MP Biomedicals) to detect secretion of pThr748 mStat1 peptide-specific IgG. The cells were cultured on the chip at 37°C for 3 hours, and IgG secreted by the cells was trapped by the coated rabbit IgG-specific antibody. After gentle washing, 10 µg/ml non-phosphorylated mouse or human Stat1-peptide was added to the chip and incubated for 30 minutes. To detect phosphorylated Stat1 peptide-specific IgG secretion, after gentle washing, 10 µg/ml biotinylated pThr748 mStat1 peptide or biotinylated pThr749 hStat1 peptide was added and incubated for 30 minutes. Then, after gentle washing, Cy3-conjugated streptavidin (Sigma) was added and incubated for 30 minutes. Finally, after gentle washing, the cells were stained with 1 µM Oregon Green (Molecular Probes) for 5 min at room temperature. Antigen-specific antibodies that were released from single cells were observed under a fluorescence microscope (BX51WI, Olympus).

Single antigen-specific antibody-secreting cells were retrieved from individual wells using a micromanipulator (TransferMan NK2, Eppendorf) fitted with capillaries (Primetech) under a fluorescence microscope, and expelled the cells into microtubes containing cell lysis solution and specific primers for the constant regions of rabbit γ and κ. cDNAs for the VH and VL fragments from the retrieved cells were amplified using the single cell 5'-RACE method (see, e.g., JP 2014-073100 A, Ozawa et al., PLoS One, 7, e52383, 2012, etc.). Briefly, reverse transcription was carried out with 15 U SuperScript III (trade name, Invitrogen) using 0.25 pmol each of Igy-RT primer (5'-GCGAGTAGAGGCCTGAGGAC-3', SEQ ID NO: 21) and Igκ-RT primer (5'-GATGCCAGTTGTTTGGGTGGT-3', SEQ ID NO: 22), 1 U RNase inhibitor (New England Biolabs), 0.5 mM each dNTPs, 5 mM DTT, 0.2% Triton-X100, and 1x 1st strand cDNA buffer solution (Invitrogen) at 55°C for 1 hour. The 3'-tailing of cDNA was performed with 20 U terminal deoxynucleotidyl transferase (Roche) using 4 mM MgCl₂, 0.5 mM dGTP, 50 mM P-K buffer solution (25 mM K₂HPO₄, 25 mM KH₂PO₄, pH 7.0), and 1 U RNase inhibitor at 37°C for 1 hour. First PCR was performed using a mixture of a dC adaptor primer (5'-AGCAGTAGCAGCAGTTCGATAACTTCGAATTCTGCAGTCGACGGTACCGCGGGC CCGGGATCCCCCCCCCCCCCDN-3', SEQ ID NO: 23) and primers for the γ and κ chain constant regions (Igγ-1st: 5'-CGAGTTCCAAGTCACGGTCA-3', SEQ ID NO: 24 and Igκ-1st: 5'-CTCCCAGGTGACGGTGACAT-3', SEQ ID NO: 25). Each product was amplified using primeSTAR DNA polymerase (TaKaRa) according to manufacturer's instructions: 30 cycles of denaturation at 94°C for 15 seconds, annealing at 55°C for 5 seconds, and extension at 72°C for 1 minute 30 seconds. Then, the resulting PCR products were diluted to 4-fold in water and 2 µl each was used for nested PCR. Nested PCR was performed with primeSTAR DNA polymerase using an adapter primer (5'-AGCAGTAGCAGCAGTTCGATAA-3') (SEQ ID NO: 26) and a nested primer specific for the y chain constant region (Igy-nest: 5'-GCCTTTGACCAAGCAGCCCAA-3', SEQ ID NO: 27) or a nested primer specific for the κ chain constant region (Igκ-nest: 5'-CGGGAAAGTATTTATTCGCCACA-3') (SEQ ID NO: 28). The obtained PCR products each were inserted into an expression vector containing cDNA of the whole constant region of a rabbit γ chain or κ chain. Thereafter, Expi293 cells were co-transfected with both the γ and κ chain expression vectors encoding whole antibody molecules using the Expi293F Expression System (Invitrogen), and the supernatants of cultured cells were collected after 3 days.

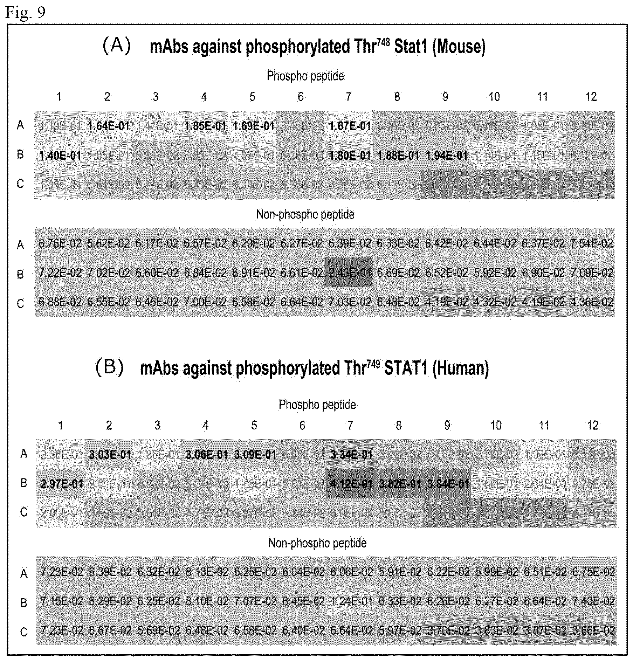

The antigenic specificity of the recombinant antibodies of 36 clones (A1 to C12) was examined by ELISA. The results are shown in Fig. 9. Panel (A) shows the measured values of color intensity in the wells immobilized with phosphorylated mouse peptide (pThr748 mStat1 peptide) (upper row) and in the wells immobilized with non-phosphorylated peptide (lower row). Panel (B) shows the measured values of color intensity in the wells immobilized with phosphorylated human peptide (pThr749 hStat1 peptide) (upper row) and in the wells immobilized with non-phosphorylated peptide (lower row). Based on these results, 8 clones (bold) that have higher specificity to both of the mouse and human phosphorylated peptides and have cross-reactivity were selected.

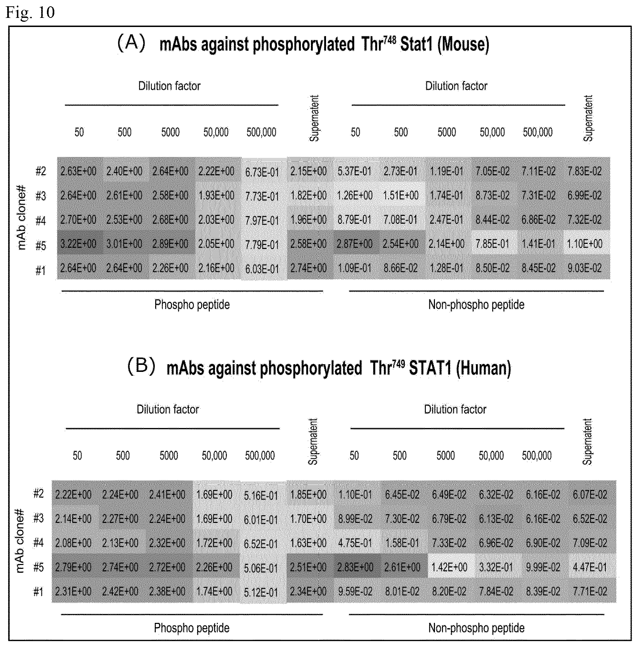

Five out of the selected eight clones were examined for phosphorylation selectivity and cross-reactivity using serial dilution samples of phosphorylated and non-phosphorylated peptides by ELISA. The results are shown in Fig. 10. Panel (A) shows the measured values of color intensity in the serial dilution samples of phosphorylated mouse peptide (pThr748 mStat1 peptide) (left) and in the serial dilution samples of non-phosphorylated peptide (right). Panel (B) shows the measured values of color intensity in the serial dilution samples of phosphorylated human peptide (pThr749 hStat1 peptide) (left) and in the serial dilution samples of non-phosphorylated peptide (right). Based on these results, clone #1 and clone #2 were selected, which had lower binding capacity to both of the mouse and human non-phosphorylated peptides.

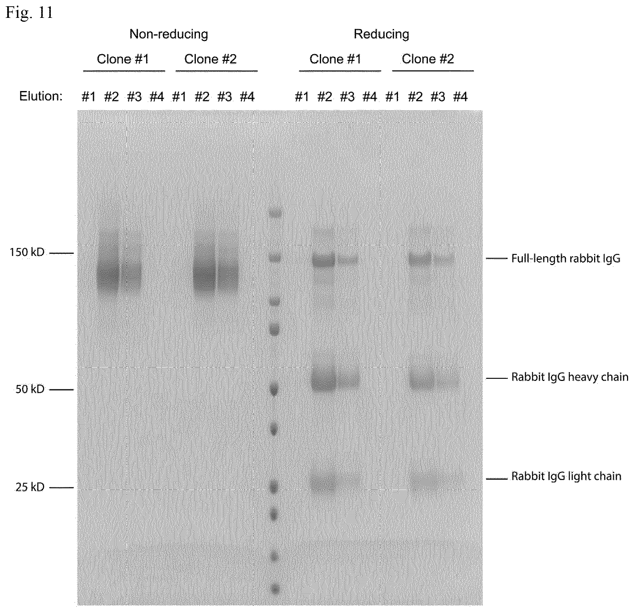

Both the y and κ chain expression vectors encoding the selected whole antibody molecules were co-introduced into HEK293T cells using Lipofectamine LTX (Thermo Fisher). HEK293T (ATCC) cells were cultured in DMEM (Nacalai Tesque) supplemented with 10% FCS (Sigma). After 48 hours of culture, supernatants containing monoclonal antibodies were collected, and the monoclonal antibodies were purified using TCS Antibody Purification Kit (Abcam) according to the manufacturer's protocol. The purified eluates of the monoclonal antibodies were run by electrophoresis under non-reducing or reducing conditions, and the bands were examined by Coomassie blue staining. The results are shown in Fig. 11.

The expression vectors as listed below were transiently introduced or co-introduced into HEK293T cells. After 24 hours of culture, whole-cell lysates were prepared and separated by SDS-PAGE for Western blotting analysis. For detection of pThr748 mStat1 and pThr749 hStat1, the prepared monoclonal antibodies were used.
- Human Flag-STAT1WT expression vector
- Human Flag-STAT1T749A expression vector
- Human Flag-STAT1WT expression vector and human HA-IKKβ expression vector
- Human Flag-STAT1T749A expression vector and human HA-IKKβ expression vector
- Mouse Flag-Stat1WT expression vector
- Mouse Flag-Stat1T748A expression vector
- Mouse Flag-Stat1WT expression vector and mouse 3xFlag-Ikkβ expression vector
- Mouse Flag-Stat1T748A expression vector and mouse 3xFlag-Ikkβ expression vector

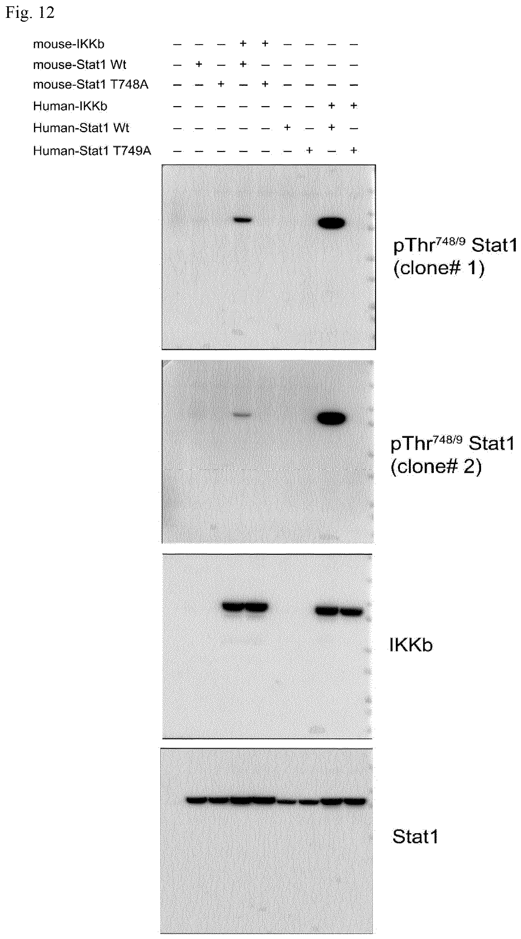

The results are shown in Fig. 12. The monoclonal antibodies from the prepared two clones were able to detect both of pThr748 mStat1 and pThr749 hStat1.

The present invention is not limited to each of the embodiments and Examples as described above, and various modifications are possible within the scope of the claims. Embodiments obtainable by appropriately combining the technical means disclosed in the different embodiments of the present invention are also included in the technical scope of the present invention. The contents of the scientific literature and the patent literature cited herein are hereby incorporated by reference in their entirety.

## Claims

1. A therapeutic agent for sepsis and/or septic shock, comprising, as an active ingredient, a compound capable of suppressing phosphorylation of threonine at position 749 in human STAT1.

2. The therapeutic agent according to claim 1, wherein the compound is a compound capable of inhibiting a protein kinase that phosphorylates threonine at position 749 in human STAT1.

3. The therapeutic agent according to claim 2, wherein the protein kinase is human TBK1 and/or human IKKβ.

4. A therapeutic agent for sepsis and/or septic shock, comprising, as an active ingredient, a compound capable of inhibiting human STAT1.

5. The therapeutic agent according to claim 4, wherein the compound is a compound capable of inhibiting a function or expression of human STAT1.

6. A method for screening for a candidate compound serving as an active ingredient of a therapeutic agent for sepsis and/or septic shock, the method comprising selecting a compound capable of suppressing phosphorylation of threonine at position 749 in human STAT1.

7. The method according to claim 6, wherein the compound is a compound capable of inhibiting a protein kinase that phosphorylates threonine at position 749 in human STAT1.

8. The method according to claim 6, wherein the compound is a compound capable of inhibiting binding between human STAT1 and a protein kinase that phosphorylates threonine at position 749 in human STAT1.

9. The method according to claim 7 or 8, wherein the protein kinase is human TBK1 and/or human IKKβ.

10. The method according to claim 6, wherein the compound is a compound capable of specifically binding to threonine at position 749 in human STAT1.

11. A method for screening for a candidate compound serving as an active ingredient of a therapeutic agent for sepsis and/or septic shock, the method comprising selecting a compound capable of inhibiting human STAT1.

12. The method according to claim 11, wherein the compound is a compound capable of inhibiting a function or expression of human STAT1.

13. A therapeutic agent for colitis, comprising, as an active ingredient, a compound capable of promoting phosphorylation of threonine at position 749 in human STAT1.

14. The therapeutic agent according to claim 13, wherein the compound is a compound capable of activating a protein kinase that phosphorylates threonine at position 749 in human STAT1.

15. The therapeutic agent according to claim 13, wherein the compound is a compound capable of promoting binding between human STAT1 and a protein kinase that phosphorylates threonine at position 749 in human STAT1.

16. The therapeutic agent according to claim 14 or 15, wherein the protein kinase is human TBK1 and/or human IKKβ.

17. A method for screening for a candidate compound serving as an active ingredient of a therapeutic agent for colitis, the method comprising selecting a compound capable of promoting phosphorylation of threonine at position 749 in human STAT1.

18. The method according to claim 17, wherein the compound is a compound capable of activating a protein kinase that phosphorylates threonine at position 749 in human STAT1.

19. The method according to claim 17, wherein the compound is a compound capable of promoting binding between human STAT1 and a protein kinase that phosphorylates threonine at position 749 in human STAT1.

20. The method according to claim 18 or 19, wherein the protein kinase is human TBK1 and/or human IKKβ.

21. The method according to claim 17, wherein the colitis is one or more diseases selected from the group consisting of inflammatory bowel disease, ulcerative colitis, Crohn disease, collagenous colitis, lymphocytic colitis, ischemic colitis, diversion colitis, Behcet disease, infectious colitis, and indeterminate colitis.

22. A therapeutic agent for systemic lupus erythematosus, comprising, as an active ingredient, a compound capable of inhibiting human STAT1.

23. The therapeutic agent according to claim 22, wherein the compound is a compound capable of inhibiting a function or expression of human STAT1.

24. A method for screening for a candidate compound serving as an active ingredient of a therapeutic agent for systemic lupus erythematosus, the method comprising selecting a compound capable of inhibiting human STAT1.

25. The method according to claim 24, wherein the compound is a compound capable of inhibiting a function or expression of human STAT1.
